# EUROPEAN PATENT SPECIFICATION

(11) **EP 2 829 231 B1**
(45) Date of publication and mention of the grant of the patent: **01.08.2018**
(21) Application number: 13764244.3
(22) Date of filing: 12.03.2013
(51) Int. Cl.: A61B 6/00, A61B 5/0245, A61B 5/026, A61B 5/0452

(54) **IMAGE-GENERATING APPARATUS**
BILDERZEUGUNGSVORRICHTUNG
APPAREIL DE PRODUCTION D'IMAGE

(30) Priority: 23.03.2012 JP 2012067186
(43) Date of publication of application: 28.01.2015
(73) Proprietor: Konica Minolta, Inc., Tokyo 100-7015 (JP)
(72) Inventor: SHIMAMURA, Kenta, Hino-shi, Tokyo 191-8511 (JP); YAMATO, Hiroshi, Hino-shi, Tokyo 191-8511 (JP); TOYAMA, Osamu, Hino-shi, Tokyo 191-8511 (JP)
(74) Representative: Hoffmann Eitle
(86) International application number: PCT/JP2013/056697
(87) International publication number: WO 2013/141067

(56) References cited:
- EP-A1- 1 970 009
- EP-A1- 2 769 675
- WO-A1-2006/137294
- WO-A1-2012/026145
- JP-A- H0 819 543
- JP-A- H01 179 078
- US-A1- 2009 148 020
- US-A1- 2010 111 380
- US-A1- 2010 208 957
- US-A1- 2011 286 652
- US-A1- 2013 156 267

## Description

### Technical Field

The present invention relates to an image-generating apparatus of a dynamic image in which a predetermined region of a human body or an animal is photographed.

### Background Art

At medical fronts, an affected area in internal organs, skeletons, and the like is photographed using an X-ray, and the like to carry out various types of tests and diagnoses. In recent years, the dynamic image, in which the motion of the affected area is captured using the X-ray, and the like, can be acquired relatively easily by applying the digital technique.

A semiconductor image sensor such as an FPD (Flat Panel Detector), and the like is used to photograph the dynamic image with respect to a subject region including a target region, and thus the diagnosis by motion analysis of the target region, and the like, which could not be performed in still image photographing and diagnosis by an X-ray photographing of the prior art, can be performed. For example, consideration is made in extracting ventilation information in a lung field from a chest X-ray dynamic image, and assisting (X-ray moving image CAD) diagnosis/treatment through quantitative analysis of the dynamic function from the change in concentration in the lung field, the motion, and the like.

Thus, in the dynamic image photographing by the FPD, a new diagnostic effect

For example, in the technique disclosed in patent document 1, when displaying the X-ray dynamic image of the lung, a graph of a distance from an apical portion of the lung to a diaphragm of each frame obtained by processing the X-ray dynamic image is displayed, and a scroll bar arranged in association with such graph is operated to display the frame of the dynamic image corresponding to the position of the scroll bar.

In the technique disclosed in patent document 2, when displaying an image stream photographed in vivo, which is not the dynamic image of the target region, a color bar for a photographing position (body tissue), temperature, and the like is displayed, where when a stripe of the color bar is pointed, the image stream is advanced to the frame corresponding to the relevant stripe.

The technique disclosed in WO 2012/026145 A1 describes a GUI-containing system that effectively utilizing kymographic images, integrates information to be used for diagnosis in a viewing system and allows accurate diagnosis even by physicians who have little experience with stethoscopes. With this diagnosis assistance system, a control unit of a diagnostic console extracts the lung field regions from the respective image frames of a plurality of image frames representing the movements of the chest, which have been transmitted from an imaging console; divides said extracted lung field regions into a plurality of sub-regions; correlates the divided sub-regions in the plurality of image frames with each other and analyses same; thereby calculating previously established characteristic quantities that represent the movement of the divided sub-regions. When the regions for display of analysis results are selected by the operating unit, the characteristic quantities for the selected sub-regions are displayed. In accordance with this system, it is made possible to provide the GUI-containing system that effectively utilizes the image imaged dynamically, integrates the information for use in the diagnosis into the visual series, and enables even the physician, who has little experience with the stethoscope, to carry out the accurate diagnosis.

### Prior Art Documents

### Patent Documents

Patent Document 1: International Patent Publication No. WO2006/137294 A1
Patent Document 2: Japanese Unexamined Patent Application Publication (Translation of PCT Application) No. 2009-508567
Patent Document 3: International Patent Publication No. WO 2012/026145A1

### Summary of Invention

### Problems to be Solved by the Invention

However, in the conventional technique of Patent Document 1, the changing amount itself in which a predetermined region, which is a diagnostic target, is time varied is displayed with a graph, and the task of searching for the effective frame is difficult.

In the conventional technique of Patent Document 2, the measurement results of the photographing position, the temperature, and the like, and the image stream are corresponded, and the analysis of the dynamic image of the target region is not mentioned at all.

In light of the foregoing, it is an object of the present invention to provide an image-generating apparatus having satisfactory workability and visibility when displaying the dynamic image of a predetermined region of a human body or an animal.

### Means for Solving the Problems

The object of the invention is achieved by the subject-matter of the independent claims. Advantageous embodiments are defined in the dependent claims. Further examples are provided for facilitating the understanding of the invention.

According to an example, it is provided an image-generating apparatus according to one aspect of the present invention including a dynamic image acquiring section that acquires a dynamic image in which a predetermined region of a human body or an animal is chronologically captured; a detecting section that detects a time variation in a physical state of the predetermined region; a diagnosis assisting information generating section that performs analysis based on the time variation in the physical state of the predetermined region detected by the detecting section, and generates a first and second analysis result based on said analysis as diagnosis assisting information, the diagnosis assisting information including a first analysis result and a second analysis result based on the analysis; a holding unit that holds the diagnosis assisting information in temporal association with the dynamic image; and a display image generating section that comprises a dynamic image display portion adapted to generate a, the displaying image is an image that includes a summary display portion that is adapted to display a progress bar (PB) corresponding to the progress of said playback image and wherein the first analysis result corresponds to the respiration phase of a right lung field and the second analysis result corresponds to the respiration phase of a left lung field and wherein the progress bar (PB) and said analysis results are displayed in an integrated manner on said progress bar and such that said first and second analysis results are displayed as parallel bars so as to be distinguishable at a glance in a time axis direction wherein said detecting section includes a predetermined region period specifying portion that is adapted to specifies specify a predetermined region period detected by said detection section, which detected and specified period becomes is a periodic time variation in a physical state of said predetermined region.

### Effects of the Invention

According to the present invention, the workability and the visibility can be enhanced when displaying the dynamic image of a predetermined region of the human

### Effects of the Invention

According to the present invention, the workability and the visibility can be enhanced when displaying the dynamic image of a predetermined region of the human body or the animal, and the diagnostic efficiency can be improved.

### Brief Description of Drawings

Fig. 1 is a view showing an overall configuration of a radiation dynamic image photographing system according to each embodiment.
Fig. 2 is a view showing a dynamic image photographed by the radiation dynamic image photographing.
Fig. 3 is a view showing a displaying image in a reference example.
Fig. 4 is a block diagram showing a functional configuration of the image-generating apparatus according to the first embodiment.
Fig. 5 is a block diagram showing the functional configuration of the image-generating apparatus according to the first embodiment.
Fig. 6 is a block diagram showing the functional configuration of the image-generating apparatus according to a sixth embodiment.
Fig. 7 is a schematic view showing a part of a waveform measured with an electrocardiograph.
Fig. 8 is a schematic view showing a set state of coordinate axes with respect to an image.
Fig. 9 is a schematic view showing the fluctuation of a cardiac wall.
Fig. 10 is a schematic view showing a fluctuation cycle of a lateral width of a heart related to a lung dynamic image in breath holding.
Fig. 11 is a view showing a display related to the blood flow.
Fig. 12 is a view of a waveform showing the time variation in a blood flow signal value of the lung blood vessel region.
Fig. 13 is a schematic view showing a contour extraction of a lung field region.
Fig. 14 is a schematic view showing a position of a feature point of the lung field region.
Fig. 15 is a view schematically showing the generation of the diagnosis assisting information of the respiration phase.
Fig. 16 is a view showing a displaying image according to the first embodiment.
Fig. 17 is a view showing a displaying image according to a second embodiment and a third embodiment.
Fig. 18 is a flowchart describing the basic operation of the image-generating apparatus realized in the first embodiment.
Fig. 19 is a block diagram showing the functional configuration of an image-generating apparatus according to a fourth embodiment.
Fig. 20 is a schematic view showing in time series the waveform data of the heart rate (blood flow).
Fig. 21 is a schematic view showing in time series the waveform data of the respiratory information.
Fig. 22 is a flowchart describing the basic operation of the image-generating apparatus realized in the fourth embodiment.
Fig. 23 is a block diagram showing a functional configuration of an image-generating apparatus according to a fifth embodiment.
Fig. 24 is a flowchart describing the basic operation of the image-generating apparatus realized in the fifth embodiment.
Fig. 25 is a view showing a joint angle in the bending and stretching of the joint.
Fig. 26 is a view schematically showing the generation of the diagnosis assisting information of the bending/stretching phase.
Fig. 27 is a view showing the displaying image of when the blood flow information is displayed as the diagnosis assisting information.
Fig. 28 is a block diagram showing some of the functional configurations of the image-generating apparatus according to the present embodiment.

### Description of Embodiments

### <1. First Embodiment>

A radiation dynamic image photographing system according to a first embodiment of the present invention will be hereinafter described.

### <1-1. Overall configuration of radiation dynamic image photographing system>

A radiation dynamic image photographing system according to a first embodiment photographs a radiation image of a subject, the subject being a human body or a body of an animal, and generates a desired displaying image.

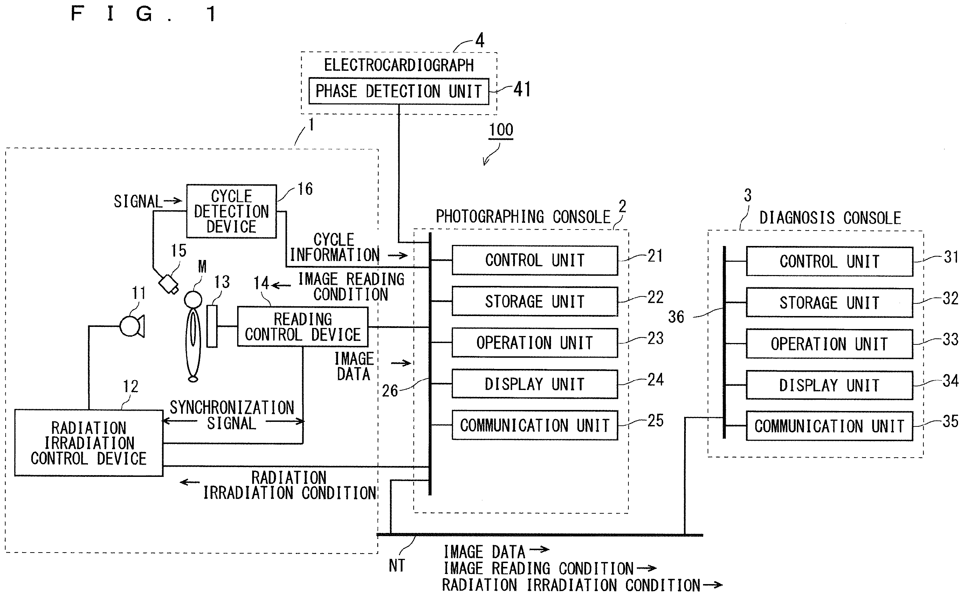

Fig. 1 is a view showing an overall configuration of the radiation dynamic image photographing system according to the first embodiment. As shown in Fig. 1, the radiation dynamic image photographing system 100 includes a photographing device 1, a photographing control device 2 (photographing console), an image-generating apparatus 3 (diagnosis console), and an electrocardiograph 4. The photographing device 1 and the electrocardiograph 4 are connected to the photographing control device 2 with a communication cable, and the like, and the photographing control device 2 and the image-generating apparatus 3 are connected by way of a communication network NT such as the LAN (Local Area Network). Each device configuring the radiation dynamic image photographing system 100 complies with a DICOM (Digital Image and Communications in Medicine) standard, and the communication among the devices is carried out in accordance with the DICOM standard.

### <1-1-1. Configuration of photographing device 1>

The photographing device 1, for example, is a device configured by an X-ray photographing device, and the like and photographs a dynamic state of the chest of a subject M involved in breathing. The dynamic state photographing is carried out by acquiring a plurality of images chronologically while repeatedly irradiating the chest of the subject M with radiation such as the X-ray, and the like. A series of images obtained by such continuous photographing is called a dynamic image. Each of the plurality of images configuring the dynamic image is called a frame image.

As shown in Fig. 1, the photographing device 1 is configured to include an irradiation unit (radiation source) 11, a radiation irradiation control device 12, an image capturing unit (radiation detection unit) 13, a reading control device 14, a cycle detection sensor 15, and a cycle detection device 16.

The irradiation unit 11 irradiates the radiation (X-ray) on the subject M in accordance with the control of the radiation irradiation control device 12. The illustrated example is a system for the human body, and the subject M corresponds to a test target. The subject M is hereinafter also referred to as "test subject".

The radiation irradiation control device 12 is connected to the photographing control device 2, and controls the irradiation unit 11 based on the radiation irradiation condition input from the photographing control device 2 to carry out radiation photographing.

The image capturing unit 13 is configured by a semiconductor image sensor such as a FPD, and the like, and converts the radiation irradiated from the irradiation unit 11 and transmitted through the test subject M to an electric signal (image information).

The reading control device 14 is connected to the photographing control device 2. The reading control device 14 controls a switching portion of each pixel of the image capturing unit 13 based on the image reading condition input from the photographing control device 2, switches the reading of the electric signal accumulated in each pixel and reads the electric signal accumulated in the image capturing unit 13 to acquire the image data. The reading control device 14 outputs the acquired image data (frame image) to the photographing control device 2. The image reading condition is, for example, a frame rate, a frame interval, a pixel size, an image size (matrix size), and the like. The frame rate is the number of frame images acquired per one second, and matches a pulse rate. The frame interval is the time from the start of acquiring operation of one frame image to the acquiring operation of the next frame image in the continuous photographing, and matches the pulse interval.

The radiation irradiation control device 12 and the reading control device 14 are connected to each other, and exchanges a synchronization signal with each other to synchronize the radiation irradiation operation and the reading operation of the image.

The cycle detection device 16 detects a respiratory cycle of the subject M and outputs the cycle information to a control unit 21 of the photographing control device 2. The cycle detection device 16 includes, for example, a cycle detection sensor 15 for detecting the motion of the chest of the subject M (respiratory cycle of the subject M) by the laser irradiation, and a timing unit (not shown) for measuring the time of the respiratory cycle detected by the cycle detection sensor 15 and outputs the same to the control unit 21.

### <1-1-2. Configuration of photographing control device 2>

The photographing control device 2 outputs the radiation irradiation condition and the image reading condition to the photographing device 1 to control the radiation photographing and the reading operation of the radiation image by the photographing device 1, and displays the dynamic image acquired by the photographing device 1 to check whether or not an image is suited for checking the positioning and diagnosis by the photographing technician.

As shown in Fig. 1, the photographing control device 2 is configured to include the control unit 21, a storage unit 22, an operation unit 23, a display unit 24, and a communication unit 25, each unit being connected by a bus 26.

The control unit 21 is configured by a CPU (Central Processing Unit), a RAM (Random Access Memory), and the like. The CPU of the control unit 21 reads out a system program and various types of processing programs stored in the storage unit 22 and develops the same in the RAM in accordance with the operation of the operation unit 23, executes various processes including a photographing control process, to be described below, according to the developed program, and intensively controls the operation of each unit of the photographing control device 2 and the operation of the photographing device 1.

The storage unit 22 is configured by a nonvolatile semiconductor memory, a hard disc, and the like. The storage unit 22 stores various types of programs to be executed by the control unit 21 and parameters necessary for the execution of the process by the program, or data such as the processing result, and the like.

The operation unit 23 is configured to include a keyboard with a cursor key, number input keys, and various types of function keys, and a pointing device such as a mouse, and the like, and outputs an instruction signal input through the key operation on the keyboard, the mouse operation, or the touch panel to the control unit 21.

The display unit 24 is configured by a monitor such as a color LCD (Liquid Crystal Display), and the like, and displays the input instruction from the operation unit 23, the data, and the like according to an instruction of a display signal input from the control unit 21.

The communication unit 25 includes a LAN adapter, a modem, a TA (Terminal Adapter), and the like, and controls the data transmission/reception with each device connected to the communication network NT.

### <1-1-3. Configuration of image-generating apparatus 3>

The image-generating apparatus 3 acquires the dynamic image transmitted from the photographing device 1 through the photographing control device 2, and displays the image for the doctor, and the like to perform radiographic image interpretation and diagnosis.

As shown in Fig. 1, the image-generating apparatus 3 is configured to include a control unit 31, a storage unit 32, an operation unit 33, a display unit 34, and a communication unit 35, each unit being connected by a bus 36.

The control unit 31 is configured by a CPU, a RAM, and the like. The CPU of the control unit 31 reads out the system program, and various types of processing programs stored in the storage unit 32 and develops the same in the RAM in accordance with the operation of the operation unit 33, executes various processes according to the developed program, and intensively controls the operation of each unit of the image-generating apparatus 3 (details will be described later).

The storage unit 32 is configured by a nonvolatile semiconductor memory, a hard disc, and the like. The storage unit 32 stores various types of programs to be executed by the control unit 31 and parameters necessary for the execution of the process by the program, or data such as the processing result, and the like. For example, the storage unit 32 stores an image generation processing program for executing an image generation process, to be described later. Such various types of programs are stored in a form of a readable program code, and the control unit 31 sequentially executes the operation according to the program code.

The operation unit 33 is configured to include a keyboard with a cursor key, number input keys, and various types of function keys, and a pointing device such as a mouse, and the like, and outputs an instruction signal input through the key operation on the keyboard, the mouse operation, or the touch panel to the control unit 31.

The display unit 34 is configured by a monitor such as a color LCD, and the like, and displays the input instruction from the operation unit 33, the data, and the displaying image to be described later, according to an instruction of a display signal input from the control unit 31.

The communication unit 35 includes a LAN adapter, a modem, a TA, and the like, and controls the data transmission/reception with each device connected to the communication network NT.

### <1-1-4. Configuration of electrocardiograph 4>

In Fig. 1, the electrocardiograph 4 is shown distant from the test subject M, but actually, each electrode terminal of the electrocardiograph 4 is attached to the test subject M to output the electrocardiographic waveform of the test subject M as a digital signal.

As shown in Fig. 1, the electrocardiograph 4 is configured to include a phase detection unit 41, the phase detection unit 41 detecting a phase of a heart rate (blood flow) of the subject M as basic information for synchronizing the photographing operation by the photographing device 1 in response to the control signal from the CPU of the control unit 21. In the present invention of the present application, the heart rate is handled as one type of blood flow. The phase detection unit 41 can also be arranged in the photographing control device 2.

### <1-2. Problems in the moving image diagnosis of a reference example>

As a premise of describing the details of the image-generating apparatus 3 in the embodiment, the problems of the moving image diagnosis in the reference example will be described.

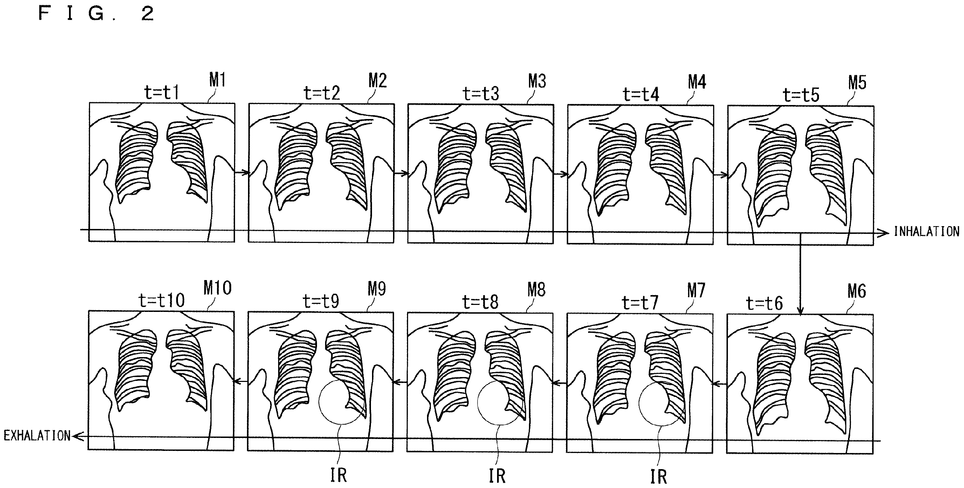

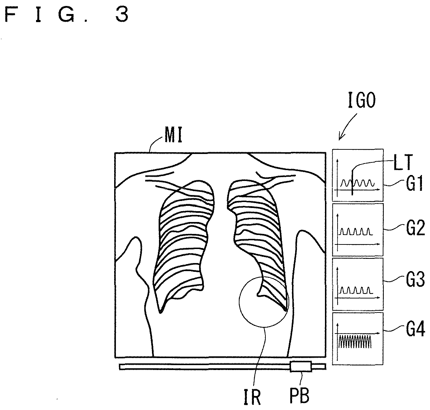

Fig. 2 shows a dynamic image photographed by the radiation dynamic image photographing with respect to the dynamic state of the chest of the test subject M involved in breathing, and Fig. 3 is a view showing a displaying image generated in the reference example. In a displaying image IGO of Fig. 3 is displayed a frame image MI, and a graph G1 showing the position of the diaphragm, a graph G2 showing the width of the rib cage, a graph G3 showing the respiratory information, a graph G4 showing the heart rate information (blood flow information) synchronized with the frame image MI are displayed along one side of the frame image MI.

As shown in Fig. 2, the frame images M1 to M10 are acquired by continuously photographing one period (details will be described later) of the respiratory cycle at a constant photographing timing. Specifically, the images photographed at the photographing timing of t = t1, t2, t3, ... t10 are corresponded to each of the frame images M1, M2, M3, ... M10. The frame images M7, M8, M9 in Fig. 2 are important frame images in the diagnosis for the user, who is a professional such as a doctor, and the like, to detect, a region IR (see Fig. 2 and Fig. 3) corresponds to a region to be diagnosed, and the phase position of the graph G1 at the current time point is shown with a line LT (see Fig. 3).

As shown in Fig. 3, in the dynamic image diagnosis according to the reference example, the desired frame image MI is searched while operating the progress bar PB corresponding to the progress of the playback of the dynamic image, and the relevant frame image MI is observed in detail to make the diagnosis. In the displaying image IGO of Fig. 3, however, the detailed diagnosis data (graph G1 and line LT herein) also need to be carefully observed although the doctor merely wants to observe the moving image, and thus the radial motion is large and the diagnostic efficiency is extremely poor.

In the actual moving image diagnosis, for example, when the moving image of the lung field is photographed at 10 Frames Per Second (fps) for 30 seconds, both the task of searching for the frame time related to the diagnosis from about 300 still images in total and the task of reproducing the dynamic images around the relevant frame time to browse and diagnose need to be carried out, which is very cumbersome. In particular, it is already a burden on the doctor to diagnose the radiographic image interpretation of 100 still images (100 people) daily, but if the radiographic image interpretation of 300 images per person is to be carried out in the moving image, the diagnosis by the radiographic image interpretation of the moving images becomes very difficult.

In particular, in the blood flow moving image diagnosis, to be described later, sufficient information is often not obtained even if only the frame for one phase (one heart rate) of the blood flow is displayed. The reasons therefor are as follows: (i) the image quality degrades as photographing is carried out at low dose to suppress exposure, (ii) whether the photographing at the optimum timing, at which the suspicion of pulmonary embolism can be determined, is carried out is unknown due to lack of fps, (iii) the desired blood flow information is not known since the way the blood flow is viewed changes at each time due to the influence of breathing, body movement, and the like. Therefore, a plurality of frames (corresponding to frame images M7, M8, M9 in the example of Fig. 2) of the relevant phase need to be searched from a plurality of periods of the blood flow of the entire moving image and diagnosed, and hence the burden of the task is very high.

Under such background, it is desired to enable the operator to selectively operate the desired frame image MI easily and to lessen the careful observation of the diagnosis data thus reducing the radial motion, and to focus the attention on the dynamic image to be originally diagnosed and continue to carefully observe the dynamic image.

In the present embodiment, the diagnosis time for the radiographic image interpretation of the moving image is reduced by generating the displaying image in which the diagnosis assisting information for grasping the desired image is easily viewed.

A specific configuration of the image-generating apparatus 3 in the first embodiment will now be described.

### <1-3. Specific configuration of image-generating apparatus 3>

The image-generating apparatus 3 of the radiation dynamic image photographing system 100 according to the first embodiment of the present invention generates a displaying image, in which the state change based on the periodic time variation in the heart, the lung, and the like (predetermined region) of the test subject M is displayed in an easily understandable manner in the time axis direction as the diagnosis assisting information, to alleviate the task of searching the desired frame image MI related to the diagnosis.

The functional configuration realized by the image-generating apparatus 3 will now be described.

### <1-3-1. Functional configuration of image-generating apparatus 3>

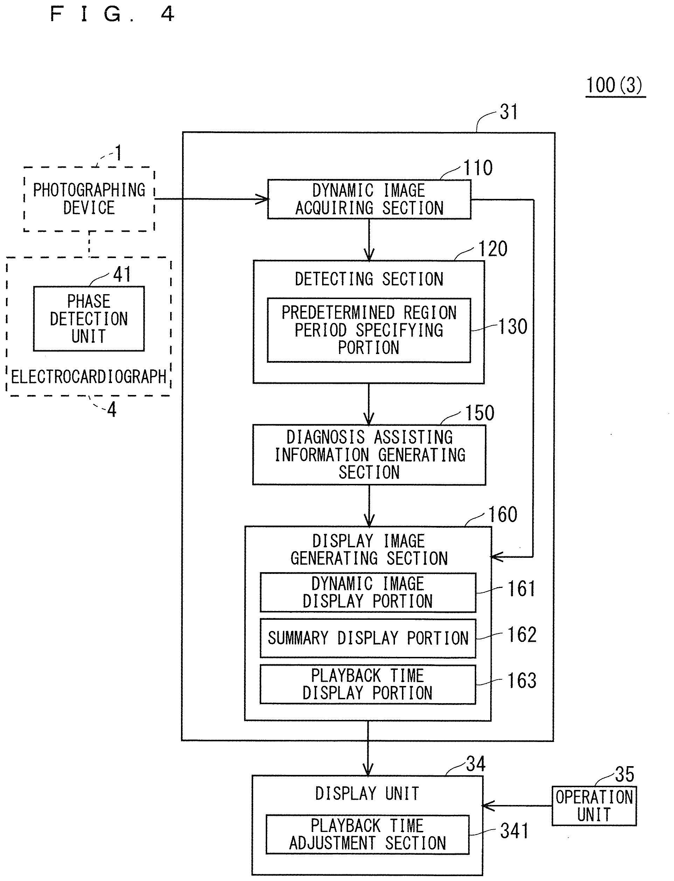

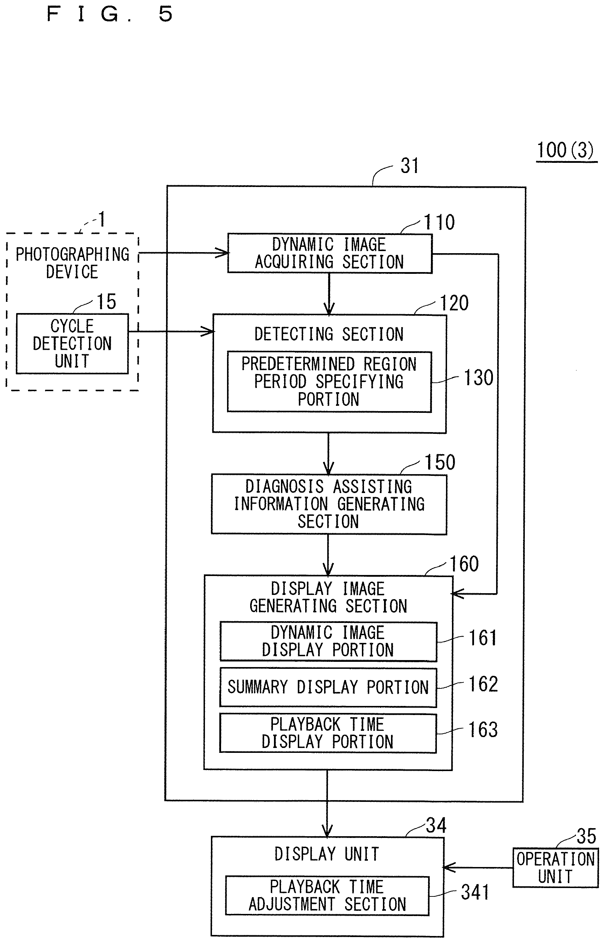

Fig. 4 and Fig. 5 are views showing the functional configurations realized by the control unit 31 when the CPU, and the like operate according to the various types of programs in the image-generating apparatus 3 of the radiation dynamic image photographing system 100, along with the other configurations. The image-generating apparatus 3 of the embodiment uses the dynamic image in which the chest including the heart and both lungs is mainly photographed.

The control unit 31 is mainly configured by a dynamic image acquiring section 110, a detecting section 120, a diagnosis assisting information generating section 150, and a display image generating section 160.

The display unit 34 displays the displaying image generated in the display image generating section 160, and is configured to include a playback time adjustment section 341 with which the user can change the playback time and refer to the displaying image.

The functional configuration of the control unit 31 as shown in Fig. 4 and Fig. 5 will be described below as being realized by the execution of the program installed in advance, but may be realized with a dedicated hardware configuration.

The specific content for each process performed by the dynamic image acquiring section 110, the detecting section 120, the diagnosis assisting information generating section 150, and the display image generating section 160 will be sequentially described with reference to Fig. 4 and Fig. 5.

### <1-3-1-1. Dynamic image acquiring section 110>

In the dynamic image acquiring section 110, the dynamic image, in which the target region (predetermined region) of a human body or an animal photographed by the reading control device 14 of the photographing device 1 is chronologically captured, is acquired. The target region is the heart region in Fig. 4 and the lung region in Fig. 5.

In Fig. 4 and Fig. 5, the photographing device 1 and the image-generating apparatus 3 are directly connected, but actually, the photographing control device 2 is interposed so that the processing data stored in the storage unit 22 of the photographing control device 2 is output to the communication unit 35 of the image-generating apparatus 3 via the communication unit 25.

### <1-3-1-2. Detecting section 120>

The detecting section 120 includes a predetermined region period specifying portion 130, and detects the time variation in the physical state of the target region (see Fig. 4 and Fig. 5). The term "physical state" referred to herein is used in a meaning including the geometric shape of the heart, the lung, and the like, the concentration of the blood flow (presence/absence of blood flow), and the like. The periodic time variation in the heart or the lung of the test subject M, that is, phase information and frequency (periodic) information of the blood flow (including heart rate) and breathing are detected. The detection of the time variation means the detection of the temporal change for the region periodic physical state such as the outer shape of the organ, the concentration of the blood flow, and the like.

In the predetermined region period specifying portion 130, the target region period (predetermined region period) to become the periodic time variation in the physical state of the target region is specified.

A first blood flow information detection method and first and second respiratory information detection methods used in the present embodiment will now be described as a method for calculating the phase information by blood flow and breathing.

First blood flow information detection method: detection result of electrocardiograph

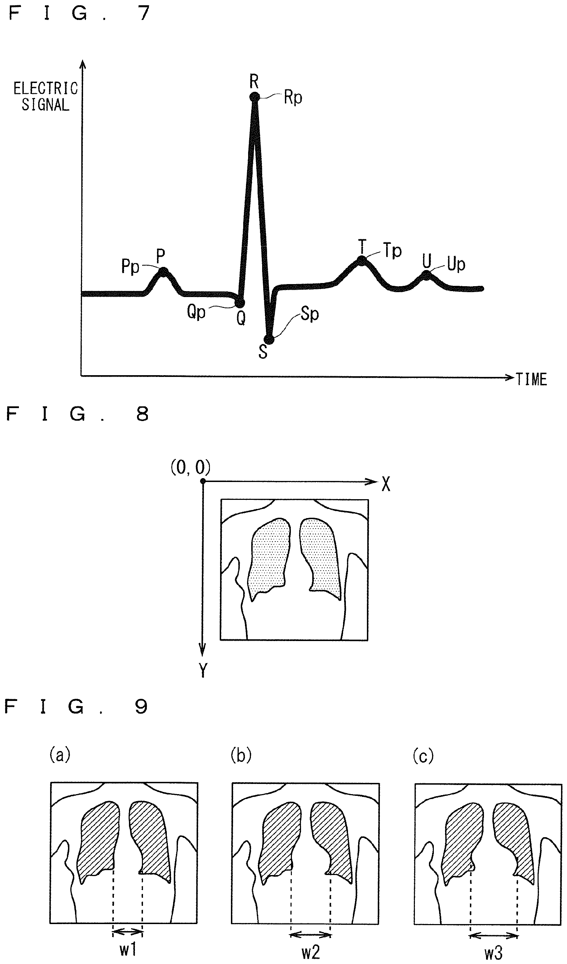

The first blood flow information detection method includes a method that uses the result acquired from the phase detection unit 41 of the electrocardiograph 4 in the detecting section 120 (predetermined region period specifying portion 130), as shown in Fig. 4. Fig. 7 is a view showing the electrocardiographic waveform of the test subject M. In Fig. 7, the horizontal axis indicates time and the vertical axis indicates the magnitude (voltage) of the electric signal, and a curve indicating the change in the electrical signal including the curves Pp, Qp, Rp, Sp, Tp, and Up, each showing the shapes of the so-called P wave, Q wave, R wave, S wave, T wave, and U wave.

In the predetermined region period specifying portion 130, the heart rate (blood flow) period is specified by analyzing the above points (Pp, Qp, Rp, Sp, Tp, and Up) based on the detection result acquired from the phase detection unit 41.

The detection operation by the phase detection unit 41 is carried out in synchronization with the image capturing operation by the photographing device 1 (see Fig. 1). In Fig. 4, the electrocardiograph 4 and the image-generating apparatus 3 are directly connected, but actually, the photographing control device 2 is interposed so that the detection data stored in the storage unit 22 of the photographing control device 2 is output to the communication unit 35 of the image-generating apparatus 3 via the communication unit 25.

Therefore, in the predetermined region period specifying portion 130, the heart rate (blood flow) period can be externally set, so that the periodic time variation of the target region can be automatically acquired.

For example, in the first blood flow information detection method, the information for generating the graph G4, which shows the blood flow information described above, can be obtained.

### First respiratory information detection method: measurement result by another equipment

The first respiratory information detection method is performed in the measurement by another equipment. For the method for measuring by another equipment, a device as described in Japanese Patent No. 3793102, for example, can be used. In addition, a method carried out by monitoring with the sensor, which is composed of laser light and a CCD camera (see for example, "A Study on respiration monitoring of a sleeping person with FG vision sensor", Hirooki Aoki, Masato Nakajima, The Institute of Electronics, Information and Communication Engineers, Society Conference, Proceedings 2001, Information, System Society Conference Report, 320-321, the 2001-08-29., etc.) or the like can be adopted.

In the present embodiment, the cycle detection sensor 15 of the cycle detection device 16 can be used in the detecting section 120 (predetermined region period specifying portion 130) as shown in Fig. 5.

Another method for detecting the respiratory cycle includes a method of detecting the motion of the chest of the subject using a breathing monitoring belt, and a method of detecting the air flow in breathing by an air speed measurement instrument, which methods can also be applied.

For example, in the first respiratory information detection method, the information for generating the graph G3, which shows the respiratory information described above, and the color bar C1, to be described later, can be obtained.

Thus, in the predetermined region period specifying portion 130, the breathing cycle can be externally set so that the periodic time variation of the target region can be automatically acquired.

### Second respiratory information detection method: area value or inter-feature point distance

In the second respiratory information detection method, an area value of the lung field portion is calculated using the photographed image acquired by the dynamic image acquiring section 110 and assumed as the respiratory information. The way of obtaining the area of the lung filed portion includes carrying out contour extraction of the lung field portion, and defining the number of pixels of the region surrounded by the contour as the lung field region. In other words, the respiratory information can be obtained by detecting the position of the diaphragm and the width of the rib cage.

For example, in the second respiratory information detection method, the information for generating the graph G1, which shows the position of the diaphragm, and the graph G2, which shows the width of the rib cage, can be obtained.

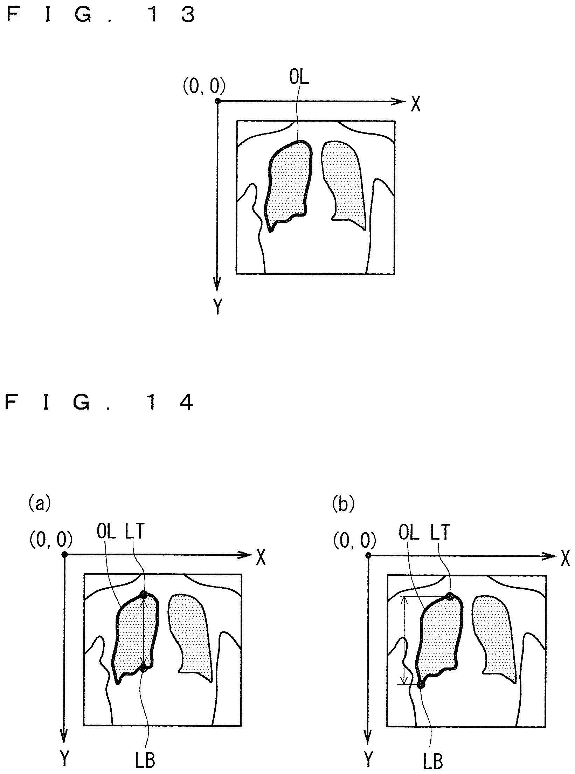

Fig. 13 is a schematic view showing the contour extraction of the lung field portion. The extraction of the lung field portion may be the extraction for each of the left and the right, or may be the extraction as the contour OL including the regions of the heart and the spine, as shown in Fig. 13. For the extracting method, the conventional technique (see e.g., "Image feature analysis and computer-aided diagnosis: Accurate determination of ribcage boundary in chest radiographs", Xin-Wei Xu and Kunio Doi, Medical Physics, Volume 22(5), May 1995, pp.617-626, etc.) can be adopted.

Thus, in the predetermined region period specifying portion 130, the contour OL of the lung field portion is extracted using the acquired photographed image, and the feature amount is detected as the area of the lung field region, the feature amount being the number of pixels in the extracted region.

In the second respiratory information detection method, the distance between the feature points of the lung field region can be calculated using the photographed image acquired by the dynamic image acquiring section 110 and assumed as the respiratory information. In other words, the extraction of the lung field portion is performed similar to the method described above, where two feature points are obtained from the extracted region, and the distance between the two points is obtained to calculate the feature amount.

Fig. 14 is a view showing the position of the feature point of the lung field region. When calculating the change in length (lung field length) from the upper end LT to the lower end LB of the lung region, an example of extraction assuming the apical portion of the lung is the upper end LT of the lung region and the intersection of a line taken in the body axis direction from the apical portion of the lung and the diaphragm is the lower end LB of the lung region is shown in part (a) of Fig. 14, and an example of extraction assuming the apical portion of the lung is the upper end LT of the lung region and the costo-transverse angle is the lower end LB of the lung region is shown in part (b) of Fig. 14.

Thus, in the predetermined region period specifying portion 130, the contour OL of the lung field region is extracted using the acquired photographed image, and the distance between the feature points is obtained from the extracted region to detect the inter-feature points and set the breathing cycle.

Therefore, in the predetermined region period specifying portion 130, the breathing cycle is detected based on the temporal change (change in the shape of the predetermined region) of the area value or the inter-feature point distance of the lung field region captured in the dynamic image, and thus the breathing cycle can be automatically acquired.

The second respiratory information detection method indirectly detects the breathing cycle compared to the first respiratory information detection method, and thus noise components are assumed to be easily contained. Thus, in the predetermined region period specifying portion 130, the breathing cycle is preferably detected using frequency analysis, and the like based on the temporal change (change in the shape of the predetermined region) of the area value or the inter-feature point distance of the lung field region captured in the dynamic image. Thus, the desired fluctuation component, from which the noise component is removed, can be automatically extracted, so that the temporal change (state in which the predetermined region temporally varies) of the area value or the inter-feature point distance of the lung field region can be more accurately grasped.

### <1-3-1-3. Diagnosis assisting information generating section 150>

In the diagnosis assisting information generating section 150, analysis is carried out based on the time variation in the physical state of the target region such as the heart, the lung, or the like detected by the detecting section 120 (predetermined region period specifying portion 130), and the analysis result is generated as the diagnosis assisting information.

The diagnosis assisting information is configured to include a first analysis result and a second analysis result based on the analysis. For example, if the target region is the lung field, the first analysis result indicates exhalation, and the second analysis result indicates inhalation. The diagnosis assisting information may include a third analysis result in addition to the first analysis result and the second analysis result. For example, if the target region is the lung field, the third analysis result may be a breath holding state.

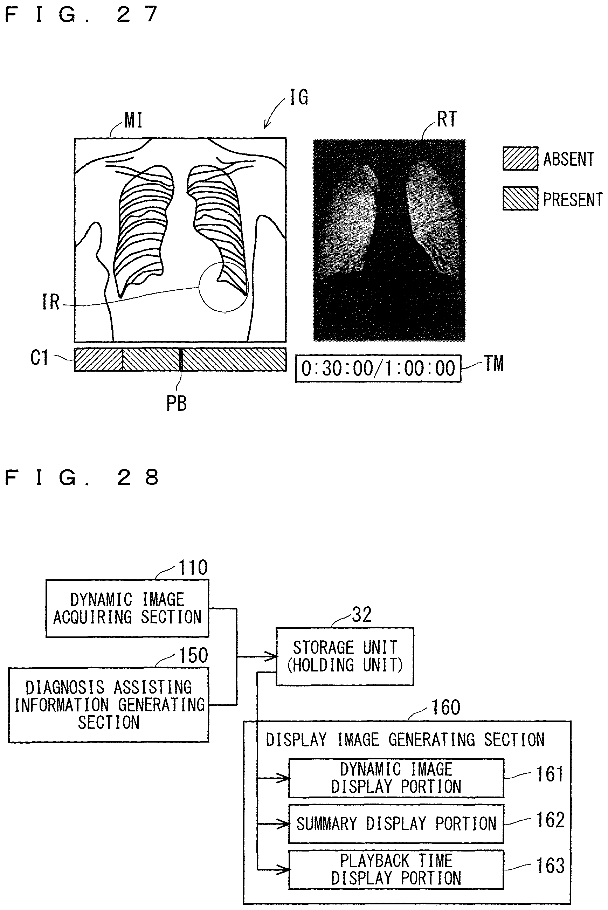

The method of holding the analysis result in the storage unit 32 (holding unit) may be a method of holding as metadata in the dynamic image, that is, a method of temporally associating the diagnosis assisting information generated in the diagnosis assisting information generating section 150 with the dynamic image (frame image MI) and holding in the holding unit 32, as shown in Fig. 28, or a method of managing as a different database. This is desirable as measurement and calculation of the image feature amount are not necessary at the time of diagnosis. The important state change information having the possibility of being displayed as the diagnosis assisting information may be exclusively held.

The diagnosis assisting information in the respiratory information and the blood flow information will now be described.

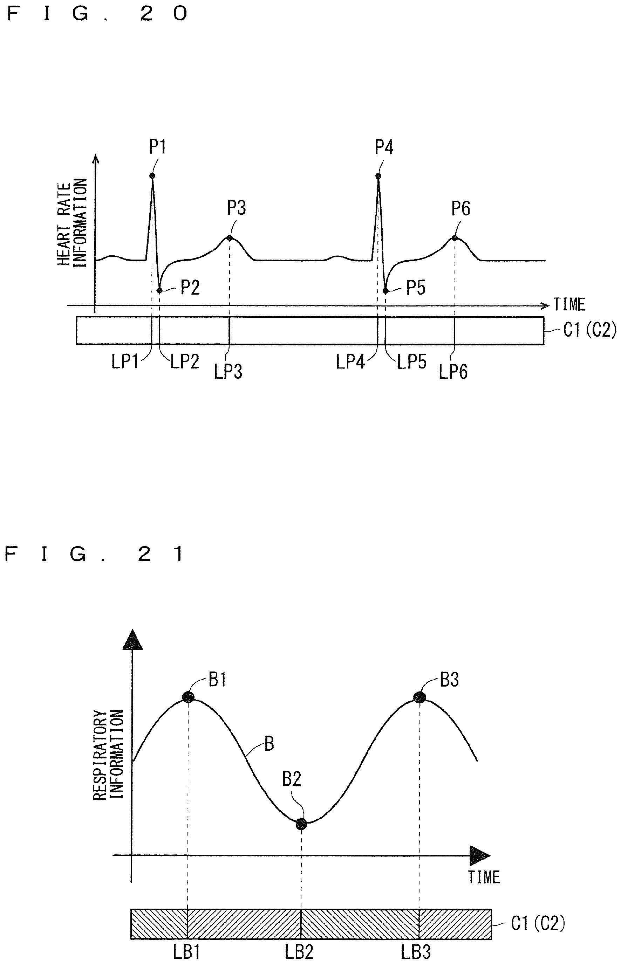

One period of the cycle (respiratory cycle) B of respiration is configured by inhalation and exhalation, and includes one exhalation and one inhalation. In the inhalation, as the diaphragm lowers and the breath is inhaled, the region of the lung field in the rib cage becomes larger. The time at which the breath is taken in to a maximum (conversion point of inhalation and exhalation) is the maximum inhalation time B1. In the exhalation, as the diaphragm rises and the breath is exhaled, the region of the lung field becomes smaller, where the time at which the breath is exhaled to a maximum (conversion point of exhalation and inhalation) is the maximum exhalation time B2 (see Fig. 21).

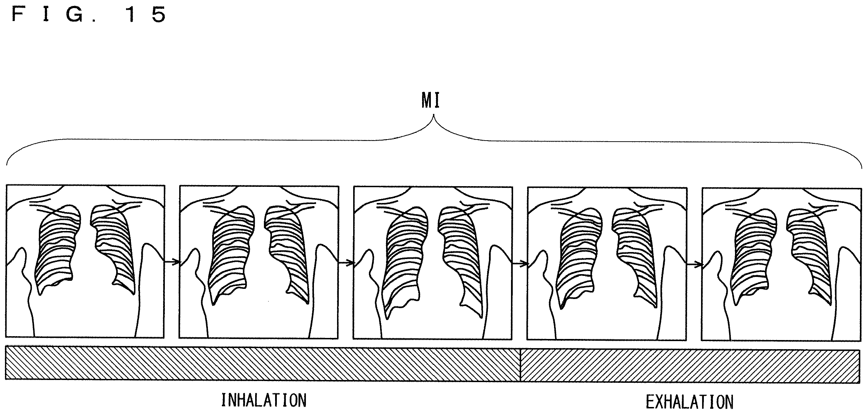

Fig. 15 is a view schematically showing the generation of the diagnosis assisting information of the respiration phase with respect to the period of the respiratory information specified in the predetermined region period specifying portion 130. As shown in Fig. 15, the first analysis result indicates the exhalation, the second analysis result indicates the inhalation, the respiration phase is corresponded to the time variation, and the diagnosis assisting information that can be distinguished in the breathing cycle is generated.

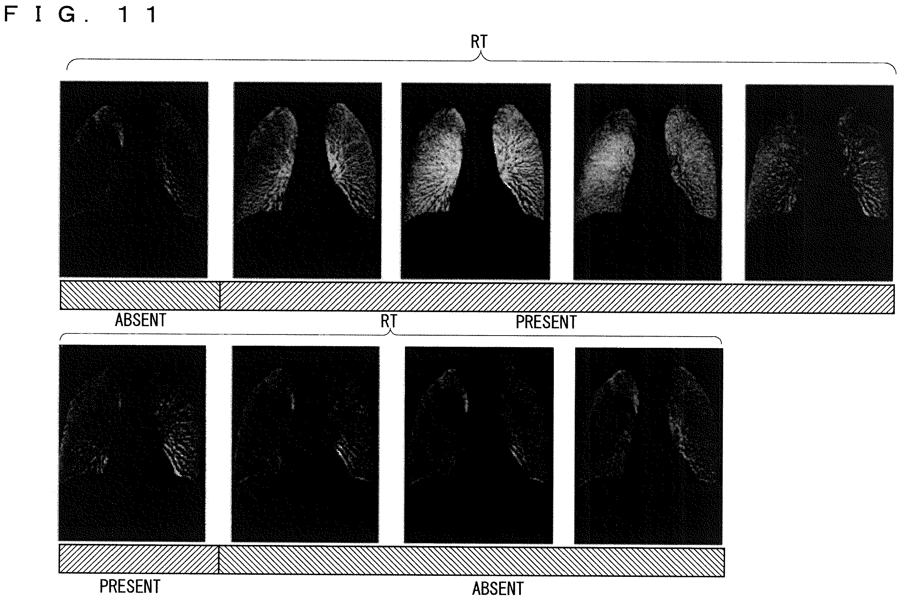

In the present embodiment, the display is made using the diagnosis assisting information in the respiratory information, but the display may be made using the diagnosis assisting information in the blood flow information (see Fig. 27). According to such case, in the blood flow information shown in Fig. 11, the first analysis result indicates "presence" of blood flow, the second analysis result indicates "absence" of blood flow, the blood flow phase is corresponded to the time variation, and the diagnosis assisting information that can be distinguished in the blood flow period is generated.

Thus, the change in the diagnosis information of the exhalation and the inhalation in the breathing cycle can be distinguished with reference to the diagnosis assisting information in the displaying image, to be described later, so that an efficient medical diagnosis can be made with respect to the state change in the lung field region of the breathing cycle. Furthermore, even in a case where the analysis result is the heart rate (blood flow) information ("systolic phase" and "diastolic phase" of the heart are first and second analysis results), an efficient medical diagnosis can be similarly made with respect to the state change in the period of the heart rate (blood flow).

The diagnosis assisting information is efficient if fixed in a case where the diagnostic content is determined in advance. For example, if the patient is suspected of pulmonary embolism, a lung blood flow phase effective to the pulmonary embolism may be adopted. Furthermore, if the patient is suspected of breathing abnormality, a respiration phase effective to the breathing diagnosis is adopted, and in addition, if some of the abnormality patterns of the respiratory system can be analyzed, a plurality of diagnosis assisting information in which the state change thereof can be recognized may be adopted.

### <1-3-1-4. Display image generating section 160>

In the display image generating section 160, the displaying image to display the frame image MI (dynamic image) and the diagnosis assisting information is generated. In other words, the phase variation in the target region and the temporally corresponding frame image MI are corresponded to generate the displaying image.

The display image generating section 160 generates the displaying image including a dynamic image display portion 161 that displays the dynamic image, a summary display portion 162 that displays the first analysis result and the second analysis result of the diagnosis assisting information so as to be distinguishable at a glance in a time axis direction, and a playback time display portion 163 that displays the playback time information corresponding to the display of the dynamic image display portion 161 (see Fig. 4 and Fig. 5). In other words, the displaying image includes the summary display portion 162, and is generated including an index, which indicates the specific position in the time axis direction of the summary display portion 162. The display image generating section 160 generates the displaying image so as to display in the dynamic image display portion 161, the dynamic image at the time point corresponding to the specific position indicated by the index. Therefore, in the displaying image of a certain time point, the dynamic image (frame image) displayed in the dynamic image display portion 161 and the specific position in the time axis direction of the summary display portion 162 indicated by the index are in a correspondence relationship. Furthermore, even if the dynamic image is displayed in a moving image mode in the dynamic image display portion 161, the diagnosis assisting information displayed in the summary display portion 162 is displayed in a still image mode, and the index of the summary display portion 162 is displayed so as to be moved in the time axis direction.

The dynamic image display portion 161 is a rectangle, and the summary display portion 162 is a long region that lies along one side of the dynamic image display portion 161, where the longitudinal direction thereof corresponds to the time axis direction of the diagnosis assisting information.

Furthermore, in the display image generating section 160, if the target region is a plurality of regions, the detecting section 120 detects the time variation in the physical state of each of the plurality of target regions, and the diagnosis assisting information generating section 150 performs the analysis based on the time variation in the physical state of each of the plurality of target regions, generates the analysis result for the plurality of target regions as the plurality of diagnosis assisting information, and the summary display portion 162 displays the plurality of diagnosis assisting information.

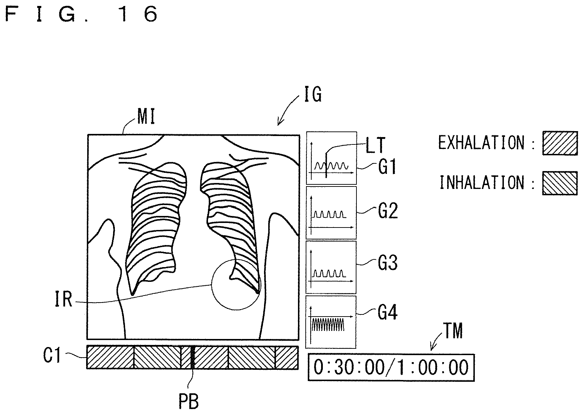

Fig. 16 is an example in which the displaying image IG generated in the display image generating section 160 is displayed on the screen of the display of the display unit 34. As shown in Fig. 16, other than the frame image MI photographed for the test subject M and the graphs G1 to G4 common with Fig. 3, the following are displayed in parallel as graphic elements in the displaying image IG.
- color bar C1 corresponding to the respiration phase of the lung field
- progress bar PB
- playback time display portion TM

In the present embodiment, the graph G3 and the color bar C1 indicating the respiratory information are information obtained from the first respiratory information detection method; and the graph G1 indicating the position of the diaphragm and the graph G2 indicating the width of the rib cage are information obtained from the second respiratory information detection method; and the graph G4 indicating the blood flow information is information obtained from the first blood flow information detection method.

In the displaying image IG shown in Fig. 16, the portion displaying the frame image MI corresponds to the dynamic image display portion 161, the portion of the color bar C1 corresponds to the summary display portion 162, and the portion of the playback time display portion TM corresponds to the playback time display portion 163.

As shown in Fig. 16, the progress bar PB and the color bar C1 are displayed in an integrated manner, as opposed to the displaying image IG0 of the reference example shown in Fig. 3.

The summary display portion 162 may adopt a display mode of displaying the first analysis result and the second analysis result such as exhalation and inhalation, and the like in different color displays (e.g., simplified display in two colors, etc.) or shading (so-called gradation). For the selection of color to be handled as the color information, the state changing amount is normalized for easy recognition when displaying the state changing amount, in particular. The method of expressing the difference in the state change includes expressing with the difference in luminance, the difference in hue, the difference in chroma, and the like, and the plurality of state changes may be expressed with luminance and hue, R-G and B-Y. The extent of the phase thus can be more clearly expressed, and the details can be grasped as necessary. Therefore, the diagnostic content of the target region can be easily visualized and the diagnostic efficiency can be further improved by referencing the displaying image IG.

Further, the playback time display portion 163 (i.e., playback time display portion TM) desirably adopts a display mode of being adjacent to the summary display portion 162 (i.e., color bar C1) so as to be recognizable. Thus, the diagnosis assisting information and the playback time at the time of playback can be visually grasped simultaneously.

Furthermore, the summary display portion 162 may include a playback time adjustment interface portion (corresponding to progress bar PB) for playback time adjustment of the dynamic image displayed in the dynamic image display portion 161 (see Fig. 4 and Fig. 5, Fig. 16).

### <1-4. Basic operation of image-generating apparatus 3>

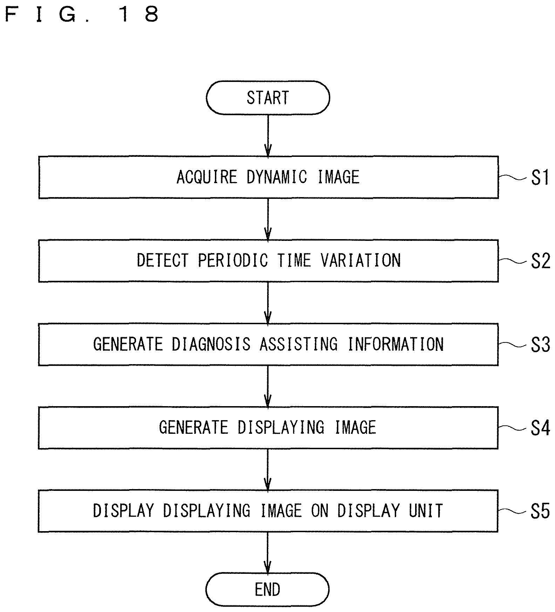

Fig. 18 is a flowchart describing the basic operation realized in the image-generating apparatus 3 according to the present embodiment. Since the individual function of each unit has already been described (see Fig. 4 and Fig. 5), the overall flow will be merely described below.

As shown in Fig. 18, first, in step S1, the dynamic image acquiring section 110 of the control unit 31 acquires the dynamic image photographed by the reading control device 14 of the photographing device 1 through the photographing control device 2.

In step S2, the detecting section 120 detects the time variation in the physical state of the heart, the lung, and the like, and the predetermined region period specifying portion 130 specifies the period of blood flow, breathing, and the like. Specifically, with regards to the time variation in the blood flow information, the detecting section 120 (predetermined region period specifying portion 130) performs the detection based on the result (first blood flow information detection method) acquired from the phase detection unit 41 of the electrocardiograph 4 (see Fig. 4). With regards to the time variation in the respiratory information, the detecting section 120 (predetermined region period specifying portion 130) performs the detection based on the result (first respiratory information detection method) acquired from the cycle detection sensor 15 (see Fig. 7 to Fig. 14) (see Fig. 5) or performs the detection based on the image feature amount (second respiratory information detection method) of the frame image M1 in the dynamic image.

In step S3, the diagnosis assisting information generating section 150 performs the analysis based on the time variation in the physical state of the heart, the lung, and the like acquired in step S2, and generates the diagnosis assisting information in which such analysis result is corresponded to the time variation (see Fig. 11, Fig. 15). The diagnosis assisting information is then temporally associated with the dynamic image, and held in the holding unit 32.

In step S4, the display image generating section 160 generates the displaying image IG for displaying the frame image MI (dynamic image) and the diagnosis assisting information held in step S3 (see Fig. 16).

Lastly, in step S5, the display image generating section 160 outputs the displaying image IG generated in step S4 to the display unit 34 to display on the monitor of the display unit 34, and then the present operation flow is terminated.

Therefore, in the image-generating apparatus 3, the dynamic image in which the target region such as the heart, the lung, and the like in the human body or the animal is chronologically captured is acquired, and the time variation in the physical state of the relevant target region is detected. Then, the analysis is carried out based on the time variation in the physical state of the target region, and the analysis result is generated as the diagnosis assisting information. The diagnosis assisting information is temporally associated and held with the dynamic image, and thereafter, the displaying image for displaying the dynamic image and the diagnosis assisting information is displayed. The diagnosis assisting information includes the first analysis result and the second analysis result based on the analysis, and the displaying image is an image including the dynamic image display portion 161 (display portion of the frame image MI in Fig. 16) that displays the dynamic image, and the summary display portion 162 (color bar C1, etc. in Fig. 16) that displays the first analysis result and the second analysis result of the diagnosis assisting information so as to be distinguishable at a glance in the time axis direction. Therefore, the professional needed to make the decision according to the "position" change of the target region that temporally varies (see line LT of Fig. 3) in the reference example, whereas in the present embodiment (including fourth and fifth embodiments to be described below), the plural types of diagnosis information (e.g., "exhalation" and "inhalation" if the analysis result is the respiratory information) in the analysis result of the diagnosis assisting information are displayed to be visually distinguishable in the time axis direction (see color bar C1 of Fig. 16), and thus the visualization having the glance property can be realized in grasping the desired displaying image. Thus, the diagnosis time for the dynamic image interpretation can be reduced, and the convenience of the user can be enhanced.

### <2. Second embodiment>

A second embodiment will now be described. The second embodiment differs from the first embodiment in that the displaying image generated by the display image generating section 160 is different. The remaining configurations are similar to the image-generating apparatus 3 of the first embodiment.

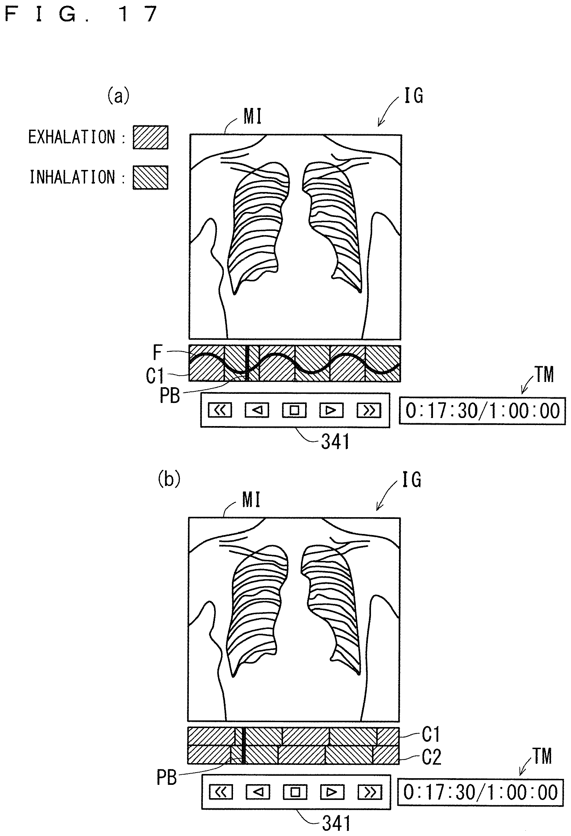

Part (a) of Fig. 17 is a view showing the displaying image IG of the second embodiment, and shows an example displayed on the screen of the display of the display unit 34. Similar to Fig. 16, the following are displayed as graphic elements other than the frame image MI photographed for the test subject M and the graphs G1 to G4 common with Fig. 3 in the displaying image IG, as shown in part (a) of Fig. 17.
- color bar C1 corresponding to the respiration phase of the lung field
- progress bar PB
- playback time display portion TM

In the displaying image IG of the second embodiment,
- waveform graph F showing the phase variation
- playback time adjustment section 341
are displayed in parallel.

In the second embodiment, the color bar C1 and the waveform graph F are information obtained from the first respiratory information detection method, and the remaining graphic elements are similar to the first embodiment.

Specifically, as shown in part (a) of Fig. 17, the displaying image IG of the second embodiment is displayed with the waveform graph F, which shows the time variation in the physical state of the target region, integrated on the color bar C1.

The summary display portion 162 includes the playback time adjustment interface portion (corresponds to progress bar PB) for playback time adjustment of the dynamic image display portion 161, and the display unit 34 includes the playback time adjustment section 341 with which the user can change the playback time to refer to the displaying image IG by using the playback time adjustment interface portion through the operation unit 35 (see part (a) of Fig. 17). Thus, the user can use the progress bar PB to change the playback time and reference the displaying image IG, so that the desired playback time of the displaying image IG can be accessed.

### <3. Third Embodiment>

A third embodiment will now be described. The third embodiment differs from the first embodiment in that the displaying image generated by the display image generating section 160 is different. Further, the displaying image in the third embodiment also differs from the displaying image in the second embodiment. The remaining configurations are similar to the image-generating apparatus 3 of the first embodiment.

Part (b) of Fig. 17 is a view showing the displaying image IG of the third embodiment, and shows an example displayed on the screen of the display of the display unit 34. Similar to Fig. 16 and part (a) of Fig. 17, the following are displayed as graphic elements other than the frame image MI photographed for the test subject M and the graphs G1 to G4 common with Fig. 3 in the displaying image IG, as shown in part (b) of Fig. 17.
- progress bar PB
- playback time display portion TM
- playback time adjustment section 341
In the displaying image IG of the third embodiment,
- color bar C1 corresponding to the respiration phase of the right lung field
- color bar C2 corresponding to the respiration phase of the left lung field
are displayed in parallel.

In the third embodiment, the color bars C1, C2 are information obtained by separately detecting the right lung field and the left lung field from the second respiratory information detection method, and the remaining graphic elements are similar to the first embodiment.

As shown in part (b) of Fig. 17, the displaying image IG of the third embodiment is displayed with the progress bar PB and the color bars C1, C2 integrated, as opposed to the displaying image IG0 of the reference example shown in Fig. 3.

As shown in part (b) of Fig. 17, for the diagnosis assisting information, for example, the color bars C1, C2 may be displayed in a proximate manner as the information obtained by temporally corresponding to each of the plurality of analysis results such as the left lung field and the right lung field, and the like. Thus, the respiration phases of the left and right lungs can be simultaneously shown, the area where the state change of the left and right lungs is different can be clearly shown and the abnormality point can be easily recognized, whereby the frame selecting operation of the moving image is facilitated.

Thus, the diagnosis assisting information is the information indicating the time variation in the plurality of analysis results corresponding to a plurality of regions, and hence the plurality of analysis results corresponding to the plurality of regions can be simultaneously visualized by referencing the displaying image IG. Furthermore, if the plurality of regions is the left lung field and the right lung field, the analysis results of each of the left lung field and the right lung field can be simultaneously visualized by referencing the displaying image IG.

Moreover, the playback time display portion 163 (i.e., playback time display portion TM) desirably adopts the display mode of being adjacent to the summary display portion 162 (i.e., color bars C1, C2) so as to be recognizable. Thus, the diagnosis assisting information and the playback time at the time of playback can be visually grasped simultaneously.

### <4. Fourth Embodiment>

As the user desires to carefully observe the moving image, the simplified display in which only the important point is displayed is effective in the frame selecting operation. Thus, in the fourth embodiment, the feature point defined under the set condition is calculated and added to the displaying image IG. The details of the feature point referred to herein will be described later, but it should be noted that it is different from the feature point in the second respiratory information detection method described above and the second blood flow information detection method to be described later.

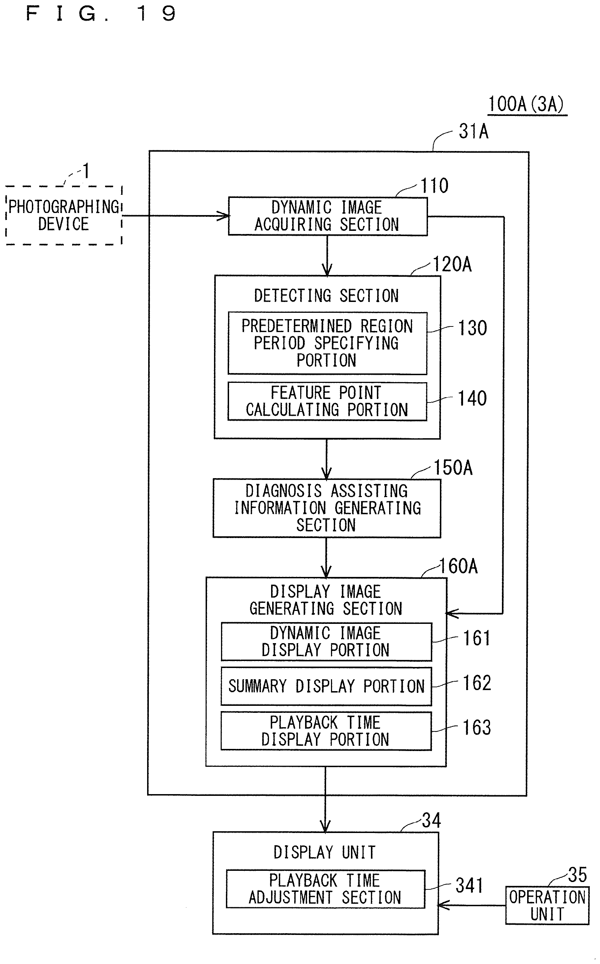

Fig. 19 is a view showing the functional configuration of a control unit 31A used in an image-generating apparatus 3A configured as the fourth embodiment of the present invention. The control unit 31A is used as a substitute of the control unit 31 (see Fig. 4) in the image-generating apparatus 3 of the first embodiment. The fourth embodiment differs from the first embodiment in that a detecting section 120A further includes a feature point calculating portion 140, and the remaining configurations are similar to the image-generating apparatus 3 of the first embodiment.

<4-1. Feature point calculating portion 140>

The feature point calculating portion 140 calculates the feature point in the time variation of the physical state of the target region. A diagnosis assisting information generating section 150A and a display image generating section 160A generate the diagnosis assisting information including the information instructing the feature point.

Fig. 20 is a schematic view showing in time series the waveform data of the blood flow (heart rate) information detected in the detecting section 120A, and Fig. 21 is a schematic view showing in time series the waveform data of the respiratory information detected in the detecting section 120A. Fig. 20 shows the result of monitoring the electrocardiographic waveform in the time direction of when the first blood flow information detection method is adopted, and Fig. 21 shows the result of calculating the temporal change in the area value or the inter-feature point distance of the lung field region when the second respiratory information detection method is adopted.

The feature points defined under the set condition include, for example, for the blood flow (heart rate) information, point P1 and point P4 (points corresponding to point Rp in Fig. 7) as the maximum points in the waveform, point P2 and point P5 (points corresponding to point Sp in Fig. 7) as the minimum points in the waveform, point P3 and point P6 (points corresponding to point Tp in Fig. 7) as the local maximum points in the waveform, as shown in Fig. 20. Similarly for the respiratory information, the feature points include point B1 and point B3 as the maximum points, and point B2 as the minimum point, as shown in Fig. 21.

In the feature point calculating portion 140, the set condition may be provided to calculate the changing point (e.g., maximum point, minimum point, local maximum point, local minimum point in first derivation or secondary derivation), for example, other than the maximum point, the minimum point, the local maximum point, and the local minimum point.

The diagnosis assisting information generating section 150A generates the displaying image IG so that the feature points calculated by the feature point calculating portion 140 are shown superimposed on the color bar C1 (C2) described above. In other words, for the blood flow (heart rate) information, point P1 and point P4 of the maximum points are displayed as lines LP1, LP4, point P2 and point P5 of the minimum points are displayed as lines LP2, LP5, and point P3 and point P6 of the local maximum points are displayed as lines LP3, LP6, respectively, as shown in Fig. 20. Similarly, for the respiratory information, point B1 and point B3 of the maximum points are displayed as lines LB1, LB3, and point B2 of the minimum point is displayed as line LB2, respectively, as shown in Fig. 21. In Fig. 20, the color bars C1 (C2) are shown blank to clarify the LP1 to LP6, but are actually displayed so that the "systolic phase" and the "diastolic phase" of the heart ("state" change of the target region) are visually distinguishable.

The diagnosis important area can be easily visualized, and the diagnostic efficiency can be further improved by clarifying the lines LP1 to LP6, LB1 to LB3 showing the feature points such as by color displaying so as to be distinguishable. If the diagnosis assisting information shows the time variation of the plurality of analysis results corresponding to the plurality of regions, for example, when showing the respiration phase of each of the left lung field and the right lung field, as shown in part (b) of Fig. 17, the line indicating the feature point is shown in a superimposed manner, so that difference in the state change is clarified and is thus useful, such as that the abnormal point (feature point) corresponding to the time variation in the state of the left lung field and the right lung field is appeared at different areas.

The frame image MI, from which diagnosis is made that abnormality is suspected in the state change, can be displayed on the color bar C1 (C2) in a distinguishable manner such as by color display by showing the feature point. Furthermore, in the first and second analysis results such as exhalation or inhalation, the abnormality can be easily found even in a situation where the reliability is low by displaying the feature point on the color bar C1 (C2) in a distinguishable manner such as by color display.

### <4-2. Basic operation of image-generating apparatus 3A>

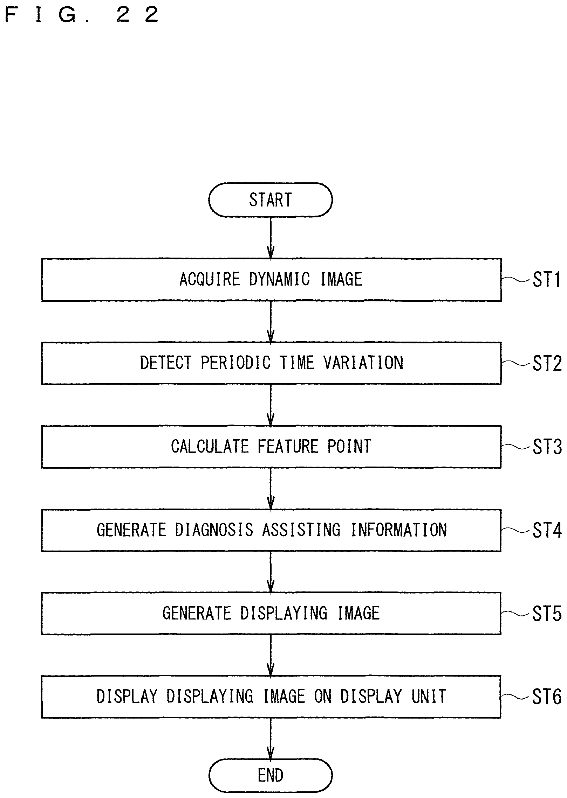

Fig. 22 is a view showing an operation flow of the image-generating apparatus 3A according to the fourth embodiment. In Fig. 22, steps ST1, ST2, ST4, ST5, and ST6 are similar to steps S1 to S5 of Fig. 18, and hence the description thereof will be omitted.

In the fourth embodiment, the feature point calculating portion 140, which did not exist in the first embodiment, is added, and thus the following steps are added.

In other words, step ST1 to step ST2 are carried out as steps similar to the first embodiment, and in step ST3, the feature point calculating portion 140 in the detecting section 120A calculates the feature point defined under the set condition in the time variation of the target region detected in step ST2, as shown in Fig. 22 (see Fig. 20 and Fig. 21).

Step ST4 to step ST5 are carried out as steps similar to the first embodiment, and lastly, in step ST6, the display image generating section 160A outputs the displaying image IG including the information instructing the feature point generated in step ST5 to the display unit 34 so as to be displayed on the monitor of the display unit 34, and then the present operation flow is terminated.

Therefore, in the image-generating apparatus 3A, the diagnosis assisting information includes the information instructing the feature point, and hence the feature point in the time variation of the target region is clarified and the diagnostic efficiency is further improved.

### <5. Fifth Embodiment>

The timing the playback time of the frame image MI satisfying the desired conditions of the user is reached or has been reached is informed to the user, which is particularly effective for the user with little experience. Thus, in the fifth embodiment, means for informing the user of the timing satisfying the desired conditions is arranged.

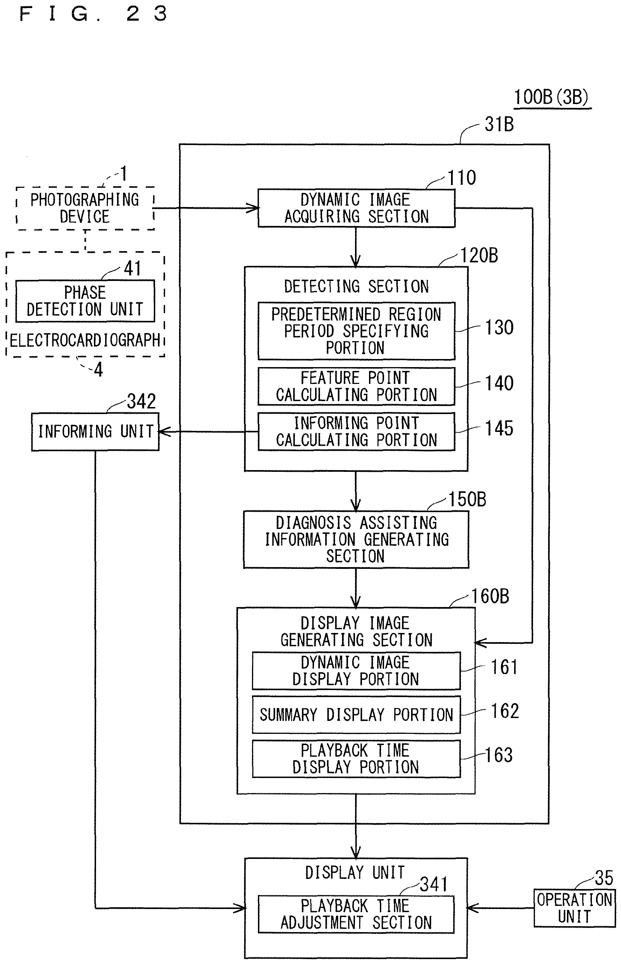

Fig. 23 is a view showing a functional configuration of a control unit 31B used in an image-generating apparatus 3B configured as the fifth embodiment of the present invention. The control unit 31B and the display unit 34B are respectively used as substitutes of the control unit 31 (31A) (see Fig. 4, Fig. 19) in the image-generating apparatus 3 (3A) of the first (fourth) embodiment. The fifth embodiment differs from the first (fourth) embodiment in that a detecting section 120B further includes an informing point calculating portion 145, and the image-generating apparatus 3B further includes an informing unit 342. The remaining configurations are similar to the image-generating apparatus 3A of the fourth embodiment.

The detecting section 120B in Fig. 23 is configured to include the feature point calculating portion 140 according to the fourth embodiment, but may not include the feature point calculating portion 140.

### <5-1. Informing point calculating portion 145 and informing unit 342>

The informing point calculating portion 145 calculates a point for informing (hereinafter referred to as "informing point") defined under the set condition desired by the user in the time variation of the target region, and outputs the same to the informing unit 342. In other words, the set condition is the condition specified by the user, and for example, if the user specifies to inform the maximum point when the time variation in the physical state of the target region is the respiratory information shown in Fig. 21, the informing point calculating portion 145 detects point P3 and point P6 as the informing points. The diagnosis assisting information generating section 150B and the display image generating section 160B generate the diagnosis assisting information including the information instructing the informing points.

When the analysis result by the diagnosis assisting information generating section 150 satisfies the set condition (predetermined condition), the informing unit 342 informs the user that the set condition is satisfied. In other words, the informing unit 342 informs the informing point detected by the informing point calculating portion 145 to the user through one of the means of visual information, auditory information, and touch information. When informing through the visual information, the informing unit 342 instructs the display unit 34B to display the visual information. Specifically, the visual information visually represents the time from the current time point to the informing point, and includes an indicator, progress bar display, display by numerical values, display by model diagram, display by periodic diagram, and the like, and preferably, is displayed on the screen in a mode enabling the user to know that the informing point is approaching from before the informing point is actually reached so that the informing point can be acquired well in advance. When informing through the auditory information, the informing unit 342 includes a buzzer, timing sound, audio, and the like. For example, the informing is made through a method of announcing the remaining seconds until the informing point with a synthetic sound, a method of ringing the buzzer at the informing point, and the like.

The progress bar PB can be operated without looking at all regardless of which one of the visual information, the auditory information, and the touch information is adopted. For example, since the user receives the informing information while performing the rewinding operation of the frame image MI according to the elapsed time, the frame image MI useful for the diagnosis can be reached and selected while carefully observing the moving image.

### <5-2. Basic operation of image-generating apparatus 3B>

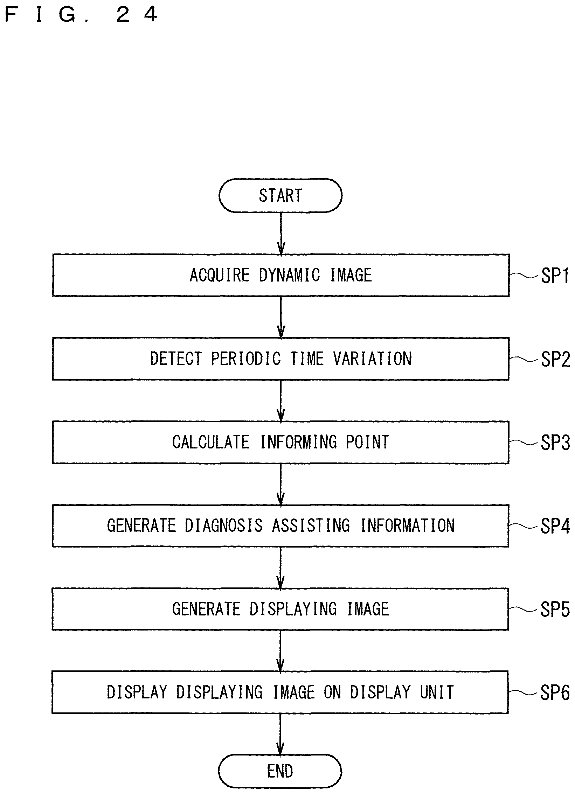

Fig. 24 is a view showing the operation flow of the image-generating apparatus 3B according to the fifth embodiment. A case in which the detecting section 120B does not include the feature point calculating portion 140 is assumed. Furthermore, in Fig. 24, steps SP1, SP2, SP4, SP5, SP6 are similar to steps S1 to S5 of Fig. 18, and hence the description thereof will be omitted.

In the fifth embodiment, the informing point calculating portion 145 and the informing unit 342, which did not exist in the first embodiment, are added, and thus the following steps are added.

In other words, step SP1 to step SP2 are carried out as steps similar to the first embodiment, and in step SP3, the informing point calculating portion 145 in the detecting section 120A calculates the informing point defined under the set condition in the time variation of the target region detected in step SP2, as shown in Fig. 24.

Step SP4 to step SP5 are carried out as steps similar to the first embodiment, and lastly, in step SP6, the display image generating section 160B outputs the displaying image IG, which takes into consideration the timing of the informing point generated in step SP5, to the display unit 34B so as to be displayed on the monitor of the display unit 34B (output by audio, touch, and the like when notifying the user of the timing of the informing point with the auditory information and the touch information), and then the present operation flow is terminated.

Therefore, in the image-generating apparatus 3B, when the analysis result of the target region satisfies the desired set condition, this is informed to the user so that even doctors, and the like with little experience in diagnosing can recognize the diagnostic content satisfying the set condition by informing.

### <6. Sixth Embodiment>

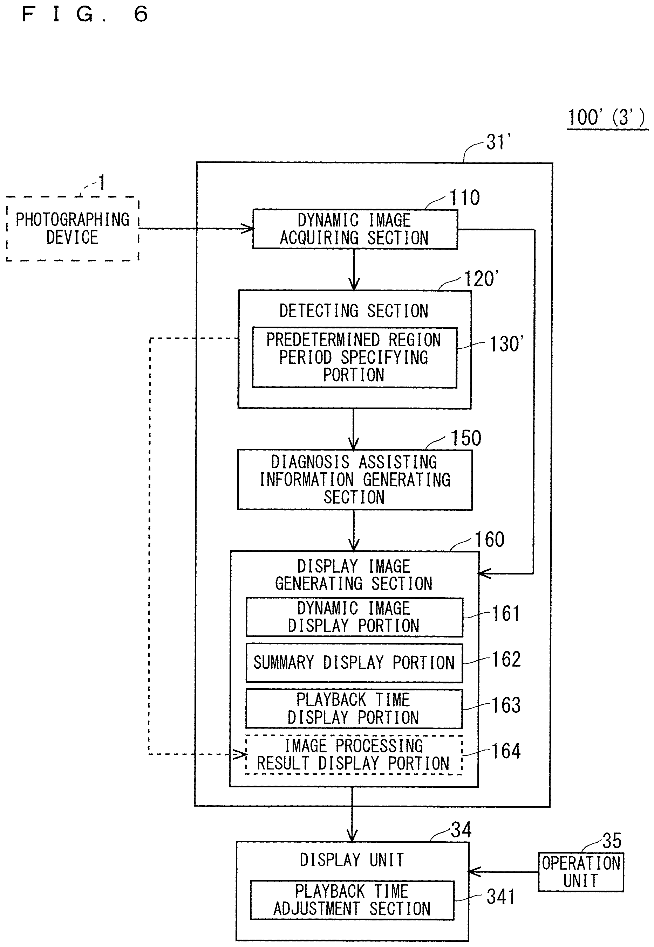

Fig. 6 is a view showing a functional configuration of a control unit 31' used in an image-generating apparatus 3' in a radiation dynamic image photographing system 100' configured as a sixth embodiment of the present invention. The control unit 31' is used as a substitute of the control unit 31 (Fig. 4) in the system 100 of the first embodiment. The sixth embodiment differs from the first embodiment in that the detection method of the blood flow information in a detecting section 120' is different. The remaining configurations are similar to the image-generating apparatus 3 of the first embodiment. The target region in the present embodiment is the heart region or the lung region. The second blood flow information detection method used in the present embodiment will now be described.

### Second blood flow information detection method: motion amount of cardiac wall

According to the second blood flow information detection method, the motion amount of the cardiac wall is calculated using the photographed image acquired by the dynamic image acquiring section 110, and assumed as the heart rate (blood flow) information in the detecting section 120' (predetermined region period specifying portion 130'), as shown in Fig. 6. In other words, the prerequisite is that the heart is also captured along with the lung, which is the target region to be photographed, in the lung dynamic image in breathing and the lung dynamic image in breath holding. Specifically, the fluctuation of the cardiac wall is detected from the lung dynamic image in breathing and the lung dynamic image in breath holding, so that the phase of the pulsation of the heart at the timing each frame image during breathing and each frame image in breath holding are photographed is detected. Therefore, the cardiac wall is detected as the phase of the pulsation of the heart.

Fig. 8 is an image coordinate plane drawn such that the value of the coordinate changes one at a time for every one pixel with a predetermined point (e.g., upper left point) as the reference point (e.g., origin), the right direction as the X-axis direction, and the downward direction as the Y-axis direction for each frame image in breathing and each frame image in breath holding.

Fig. 9 is a schematic view showing the fluctuation of the cardiac wall captured in the lung dynamic image in breath holding. As an example of the fluctuation of the cardiac wall, the fluctuation of the lateral width of the heart is adopted. In parts (a) to (c) of Fig. 9, a state in which the lateral width of the heart increases from wl to w3, which is the motion of the cardiac wall, in the course of heart dilation is shown.

Thus, in the predetermined region period specifying portion 130, the lateral width of the heart is detected from each frame image in breathing and each frame image in breath holding to set the heart rate (blood flow) period. Specifically, the method of detecting the lateral width of the heart includes, for example, a method of detecting the contour of the heart, and the like. Various known methods can be adopted for the method of detecting the contour of the heart, and for example, a method of aligning the feature point in the X-ray image and the feature point of the heart model using the model (heart model) showing the shape of the heart to detect the contour of the heart (see e.g., "Image feature analysis and computer-aided diagnosis in digital radiography: Automated analysis of sizes of heart and lung in chest images", Nobuyuki Nakamori et al., Medical Physics, Volume 17, Issue 3, May, 1990, pp.342-350, etc.), and the like can be adopted.

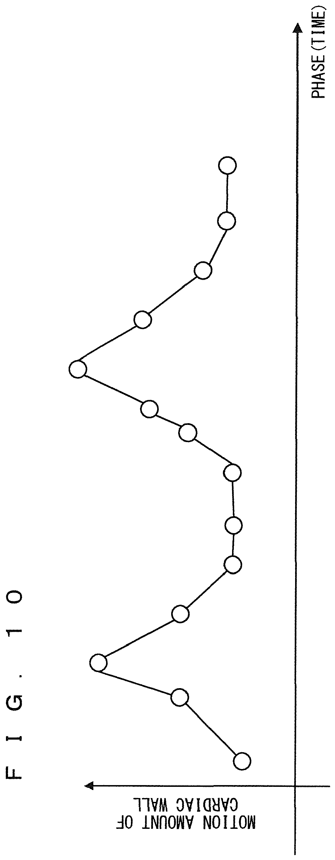

Fig. 10 is a schematic view showing a relationship of the photographed time and the lateral width of the heart for a plurality of frame images in breath holding, which configure the lung dynamic image in breath holding. In Fig. 10, the horizontal axis indicates the time, the vertical axis indicates the lateral width of the heart, and the circle mark indicates the value of the detected lateral width of the heart.

Assuming the lateral width of the heart captured at time t is Hwt and the lateral width of the heart captured at time t + 1 is Hwt + 1, the frame image in breath holding captured at time t is classified to the time of dilation of the heart when (Hwt + 1 - Hwt) ≥ 0 is satisfied, and the frame image in breath holding captured at time t is classified to the time of contraction of the heart when (Hwt + 1 - Hwt) < 0 is satisfied.

Therefore, in the predetermined region period specifying portion 130', the heart rate (blood flow) period is detected based on the motion of the cardiac wall (change in the shape of the predetermined region) captured in the dynamic image, and thus the relevant period can be automatically acquired.

The second blood flow information detection method indirectly detects the blood flow period compared to the first blood flow information detection method, and thus noise components are assumed to be easily contained. Thus, in the predetermined region period specifying portion 130', the blood flow period is preferably detected using the frequency analysis, and the like based on the motion of the cardiac wall (change in the shape of the predetermined region) captured in the dynamic image. Thus, the desired fluctuation component, from which the noise component is removed, can be automatically extracted, so that the motion amount of the cardiac wall (state in which the predetermined region temporally varies) can be more accurately grasped.

### <7. Seventh Embodiment>

A seventh embodiment will now be described. The seventh embodiment differs from the first embodiment in that the detection method of the blood flow information in the detecting section 120 is different. The remaining configurations are similar to the image-generating apparatus 3 of the first embodiment. The detection method of the blood flow information in the seventh embodiment differs from the detection method of the blood flow information in the sixth embodiment, but is common in that the blood flow information (time variation in the physical state of the target region) is detected based on the dynamic image as shown in Fig. 6. The third blood flow information detection method used in the present embodiment will be described below.

Third blood flow information detection method: blood flow phase analysis

In the third blood flow information detection method, the blood flow phase analysis is carried out using the photographed image acquired by the dynamic image acquiring section 110 to obtain the blood flow information. The blood flow phase is the phase information indicating the presence or absence of the blood flow corresponding to the position where the blood is flowing. With regards to the blood flow phase analyzing process (blood flow information generating process) used in the present invention, for example, "Japanese Patent Application No. 2011-115601 (filing date: May 24, 2011) filed by the applicant of the present application can be adopted.

Fig. 11 is a view showing the analysis result of time-space variation involved in the presence or absence of the blood flow of the entire lung. Generally, since the lung field blood vessel dilates when the blood is rapidly discharged from the right ventricle through the aorta due to the contraction of the heart, such dilation is extracted by analyzing the dynamic image and output as the diagnosis assisting information associated with the presence or absence of the blood flow of the entire lung in the blood flow analysis. In other words, when the blood vessel is dilated in the lung field, the radiation transmission amount of the region where the lung blood vessel dilated relatively reduces greatly than the radiation transmission amount that transmitted the lung field (lung alveolus) region, and hence the output signal value of the radiation detection unit 13 corresponding to such region lowers. The dilation of the lung blood vessel corresponding to the pulsation of the heart is propagated from the artery in the vicinity of the heart to the periphery. The pixel unit of the radiation detection unit 13 among the series of frame images MI configuring the dynamic image, or the small region unit (pixel block unit) including a plurality of pixels are corresponded to each other, the frame image MI in which the signal value is the lowest is obtained for every pixel unit or small region unit, and the corresponding region of the frame image MI is colored as a signal indicating the timing the lung blood vessel is dilated by the blood flow. As shown in Fig. 11, the series of frame images MI after the coloring are sequentially displayed on the display unit 34, so that the state of the blood flow can be visualized by the doctor, and the like. The white area shown in Fig. 11 is actually colored in red, and the like.

In each pixel (small region), the signal (referred to as blood flow signal) indicating the timing at which the lung blood vessel is dilated by the blood flow can be acquired by obtaining the local minimum value of the waveform (referred to as output signal waveform) indicating the time variation in the signal value of the relevant pixel (small region). The blood flow signal appears at the same interval as the pulsation period of the heart, but if an abnormal area such as arrhythmia, and the like exists, the local minimum value sometimes appears irrespective of the dilation of the blood vessel involved in the blood flow at an interval different from the pulsation period of the heart. Thus, in the third blood flow information detection method, the blood flow signal can be accurately extracted by obtaining the correlation coefficient of the pulsation signal waveform showing the pulsation of the heart and the output signal waveform of each small region.

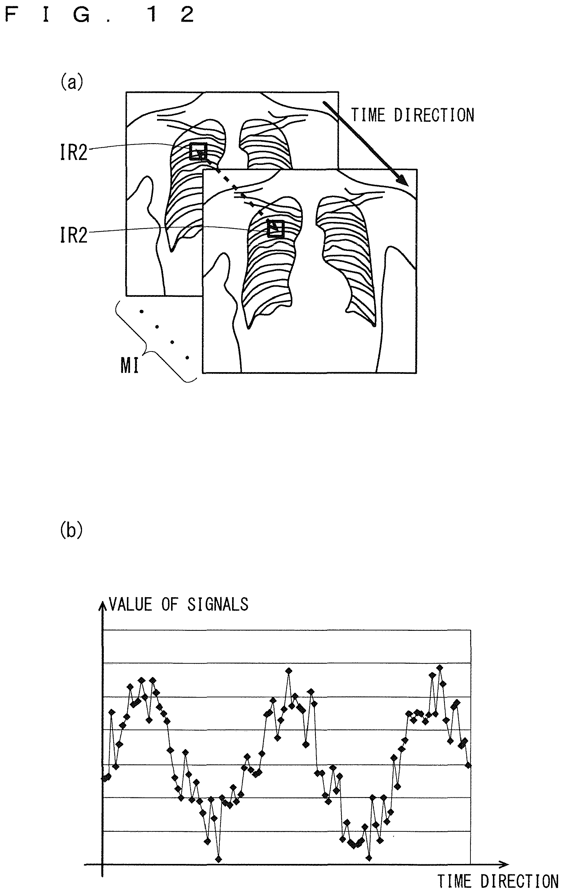

The outline of the output signal waveform of the lung blood vessel region and the blood flow signal extracting method will now be described for the detection of the blood flow information in the lung blood vessel region. Fig. 12 is a view illustrating the waveform showing the time variation in the blood flow signal value of the lung blood vessel region. In part (a) of Fig. 12, the position of the lung blood vessel region IR2 corresponding to the region to be diagnosed is shown in the chronologically acquired series of frame images MI, and in part (b) of Fig. 12, a graph in which the signal value (representative value) of the lung blood vessel region IR2 of each frame image MI is plotted on the coordinate space having the horizontal axis as the elapsed time (frame number) from the start of photographing of the dynamic image and the vertical axis as the signal value (representative value) in the lung blood vessel region IR2 is shown.

In the graph of part (b) of Fig. 12, the phases of the breathing and the blood flow are in a coexisting state, and hence the respective influences of the breathing and the blood flow are eliminated through the following filtering process. In other words, in the filtering process, the low frequency signal variation by the breathing, and the like is removed, and the time variation in the signal value by the blood flow is extracted. For example, with respect to the time variation in the signal value for every small region, the high pass filtering is carried out at the low pass cut-off frequency of 0.7 Hz in a quiet breathing image group, and at the low pass cut-off frequency of 0.5 Hz in the deep breathing image group. Alternatively, to further remove the high frequency noise component, the filtering may be carried out by the band pass filter that also shields the high frequency at the high pass cut-off frequency of 2.5 Hz.

The cut-off frequency is preferably optimized for every photographed dynamic image rather than being a fixed value. For example, the time of systolic phase and the time of diastolic phase (relaxation phase) of the heart are calculated from the signal variation in the heart region of the series of frame images MI (see part (a) of Fig. 12) (see second blood flow information detection method). The value obtained by multiplying a predetermined coefficient to the inverse number of the time of diastolic phase is set as the low pass cut-off frequency for shielding the low frequency in the high pass filter or the band pass filter, and the value obtained by multiplying a predetermined coefficient to the inverse number of the time of systolic phase is set as the high pass cut-off frequency for shielding the high frequency in the case of the band pass filter. Further, the low pass cut-off frequency may take into consideration the frequency component by breathing, and analyze the value of the position of the diaphragm and the area value or the inter-feature point distance of the lung field region from the series of frame images (see second respiratory information detection method to be described later), detect the frame image MI to become the quiet exhalation position and the quiet inhalation position in the case of the quiet ventilation, obtain the time for the inhalation phase from the number of frames between the frame of the quiet exhalation position and the frame of the next quiet inhalation position, and set the inverse number thereof and the value obtained by multiplying a predetermined coefficient to an average value of the time of the diastolic phase as the low pass cut-off frequency. In the case of the quiet ventilation, the automatically set cut-off frequency is preferably limited to 0.2 to 1.0 Hz for the low pass cut-off frequency and to 2.0 Hz and higher for the high pass cut-off frequency. The vital sign such as the breathing rate, number of pulses, and the like (see first blood flow information detection method and first respiratory information detection method to be described later) in one minute at the time of resting, which are separately measured, may be input as the patient information, and the cut-off frequency may be calculated from such values. For example, the breathing rate in one minute input as the patient information may be converted to the breathing rate in one second, and a value obtained by multiplying a predetermined coefficient to such breathing rate may be set as the low pass cut-off frequency. The input number of pulses in one minute may be converted to the number of pulses in one second, and the value obtained by multiplying a predetermined coefficient to the breathing rate in one second may be set as the high pass cut-off frequency. Further, the value obtained by multiplying the predetermined coefficient to the average value of the breathing rate in one second and the number of heart beat in one second may be set as the low pass cut-off frequency.

After the blood flow phase analyzing process described above, the blood flow period is specified based on the blood flow phase variation (change in the state of the predetermined region) captured in the dynamic image in the predetermined region period specifying portion of the present embodiment, and hence the blood flow period can be automatically acquired.

### <8. Variant>

The embodiments of the present invention have been described above, but the present invention is not limited to the above embodiments, and various modifications can be made.

In the present embodiment, the image-generating apparatus 3, 3', 3A, 3B is described according to each embodiment so as to be individually implemented, but the individual functions may be mutually combined as long as they do not contradict each other.

In the present invention, the region in which the physical state periodically temporally varies among the portions to be photographed in the body is the target of phase detection, but this is not limited to the heart, the lung, and the like, and may be other organs that perform involuntary movement such as vermiculation, and the like, or may be a region that performs voluntary movement such as muscles, joints, and the like. In the latter case, the dynamic state photographing is carried out while causing the test subject to repeatedly perform the same movement.

In other words, in the present embodiment, the respiratory information and the blood flow information in the chest photograph are assumed as the target, but for example, the bending/stretching direction information, and the like of the joint in the joint photograph may be assumed as the target.

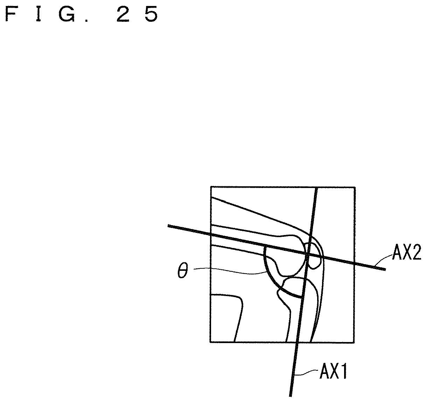

Fig. 25 is a view showing the joint angle in the bending and stretching of the joint. As shown in Fig. 25, the bending/stretching direction information is calculated from the movement of the joint angle θ by the detecting section 120 (120A, 120B). Specifically, the contour extracting method of the second respiratory information detection method, the threshold processing, and the like are adopted to extract the contour region of the bone. The axes AX1, AX2 are extracted from the contour region, and the bending/stretching direction is calculated from the fluctuation of the angle θ at which the two axes AX1, AX2 intersect. The axes AX1, AX2 may pass through the center of the contour region, or may be lines that lie along the edge of the contour region.

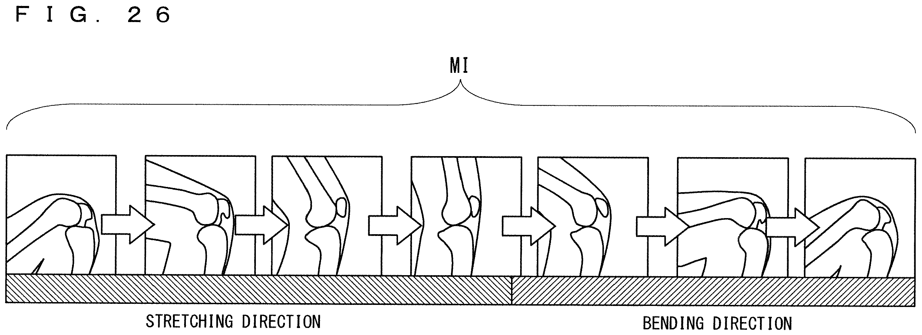

Fig. 26 is a view schematically showing the generation of the diagnosis assisting information of the bending/stretching phase with respect to the period of the bending/stretching direction information specified in the predetermined region period specifying portion 130. As shown in Fig. 26, the first and second analysis results indicate the stretching direction and the bending direction, the bending/stretching phase corresponds to the time variation, and the diagnosis assisting information that can be distinguished similar to Fig. 11 and Fig. 15 is also generated in the bending/stretching phase period.

Thus, the change in the stretching direction and the bending direction in the bending/stretching period can be distinguished with reference to the diagnosis assisting information in the displaying image IG, and thus the efficient medical diagnosis can be made with respect to the state change in the joint of the bending/stretching period.

In the image analysis of the detecting section 120, the feature point calculating portion 140, and the informing point calculating portion 145, the target region of the image analysis of the regions of the frame image MI can be appropriately set. Thus, the calculation time required for the image analysis can be shortened.

In the present embodiment, a case of displaying the respiratory information in the summary display portion 162 (color bars C1, C2) in the displaying image IG as the analysis result of the diagnosis assisting information is shown, as shown in Fig. 16 and Fig. 17, but the blood flow information shown in Fig. 11 may be displayed. The display image generating section 160 may generate the displaying image IG so as to not only display the blood flow phase on the color bar C1 (C2), but to display the image processing result RT (see Fig. 11) by the third blood flow information detection method (blood flow phase analysis) described above superimposed or proximate to the frame image MI.

Fig. 27 shows the displaying image IG of when displaying the blood flow information as the analysis result of the diagnosis assisting information. Upon generating the displaying image IG, the detecting section 120 outputs the image processing result RT (see Fig. 11) related to the presence or absence of the blood flow of the target region based on the frame image MI (dynamic image), and temporally associates the same with the dynamic image and holds the same in the holding unit 32, as shown in Fig. 4. The display image generating section 160 generates the displaying image IG so as to display the image processing result RT in synchronization with the dynamic image.

The displaying image IG desirably includes an image processing result display portion 164 (broken line area in Fig. 4) for displaying the image processing result RT as a visually distinguishable display content. In other words, as shown in Fig. 27, the image processing result display portion 164 (i.e., display portion of the image processing result RT) desirably adopts a display mode of being superimposed (not shown) or brought proximate (see Fig. 27) to the dynamic image display portion 161 (i.e., display portion of the frame image MI) so as to be distinguishable. Thus, the frame image MI, the diagnosis assisting information, and the image processing result RT can be visually grasped simultaneously, and a more efficient medical diagnosis can be made.

When suspected of pulmonary embolism, the suspicious area can be checked in detail and diagnosed. For example, the blood flow phase situation in the lung field is determined on the color bar C1, and the blood flow situation of the lung blood vessel having the possibility of pulmonary embolism can be carefully observed in the image processing result RT (frame image MI in the case of superimposed display). Furthermore, if whether the position having the possibility of pulmonary embolism is the lung blood vessel near the heart or the lung blood vessel at the periphery is known, the user can adjust the playback time the user desires to see in the diagnosis from the phase information using the progress bar PB.

As an example in which the diagnosis assisting information indicates the time variation of a plurality of analysis results corresponding to a plurality of regions, the phases of the right lung field and the left lung field are respectively shown on the color bars C1, C2 in part (b) of Fig. 17, but the phases of the respiratory information and the blood flow information may be simultaneously displayed. This is because the diagnosis of the blood flow is most visually recognized at the state of the respiration phase being the maximum inhalation as the lung becomes the largest.

As described above, the time variation of only the target region may be detected by the detecting section 120, and the phase information of such area of interest may be displayed on the color bar C1 (C2). This is because if the location suspected of pulmonary embolism is definite, the display of the phase information limited to the relevant area is desirable.

Furthermore, the phase of the blood flow may be expressed with not only the two phases by the presence or absence of the blood flow of the specific region, and the area may divided to the main blood vessel region and the peripheral blood vessel region of the lung field so that the phase may be expressed with three phases depending on which region the blood flow exists. Thus, the specification and selection of the playback time corresponding to in which of the main blood vessel or the peripheral blood vessel the pulmonary embolism is suspected are facilitated.

The displaying image IG generated by the display image generating section 160, 160A, 160B is not limited to the examples of the present embodiment. In other words, the displaying image IG can be generated in correspondence with various diagnostic standpoints by enabling user customization. For example, when the user clicks on the specific motion (state change) information displayed in a graph and performs the frame selecting operation, the color display may be switching changed with such motion information as the target. Furthermore, a certain pixel region may be specified so that the motion may be analyzed for the time direction of the relevant pixel region, and the color display may be switched according to the analysis result.

In the present embodiment, when expressing the position of the frame image MI by moving the progress bar PB in the horizontal direction, the color of the color bar C1 (C2) is also changed in correspondence with the horizontal coordinate along the progress bar PB (see Fig. 16 and Fig. 17), but this is not the sole case. For example, when expressing the position of the frame image MI by moving the progress bar PB in the vertical direction or the rotation direction, the color of the color bar C1 (C2) may also be changed along such direction.

The subject is not only the human body, and may be the body of an animal.
- 1 :: photographing device
- 2 :: photographing control device
- 3, 3', 3A, 3B :: image-generating apparatus
- 4 :: electrocardiograph
- 31, 31', 31A, 31B :: control unit
- 34, 34B : display unit 41 :: phase detection unit
- 100, 100', 100A, 100B :: radiation dynamic image photographing system

- 110 :: dynamic image acquiring section
- 120 :: detecting section
- 130, 130' :: predetermined region period specifying portion
- 140 :: feature point calculating portion
- 145 :: informing point calculating portion
- 150, 150A, 150B :: diagnosis assisting information generating section

- 160, 160A, 160B :: display image generating section
- 161: dynamic image display portion
- 162: summary display portion
- 163: playback time display portion
- 164: image processing result display portion

- 341: playback time adjustment section
- 342: informing unit
- C1, C2: color bar
- PB: progress bar
- M: subject (test subject)
- MI: frame image
- TM: playback time display portion
- RT: image processing result

## Claims

1. An image-generating apparatus (3) comprising:
a dynamic image acquiring section (110) that acquires a dynamic image (MI) in which a predetermined region of a human body or an animal is chronologically captured;
a detecting section (120) that detects a time variation in a physical state of said predetermined region;
a diagnosis assisting information generating section (150) that performs analysis based on the time variation in the physical state of said predetermined region detected by said detecting section (120), and generates a first and second analysis result based on said analysis as diagnosis assisting information, said diagnosis assisting information including a first analysis result and a second analysis result based on said analysis,
a holding unit (32) that holds said diagnosis assisting information in temporal association with said dynamic image (MI); and
a display image generating section (160) that comprises a dynamic image display portion adapted to generate a playback image adapted to play back said dynamic image (MI), said displaying image (IG) is an image that includes a summary display portion that is adapted to display a progress bar (PB) corresponding to the progress of said playback image and wherein the first analysis result corresponds to the respiration phase of a right lung field and the second analysis result corresponds to the respiration phase of a left lung field and wherein the progress bar (PB) and said analysis results are displayed in an integrated manner on said progress bar and such that said first and second analysis results are displayed as parallel bars so as to be distinguishable at a glance in a time axis direction,
wherein said detecting section (120) includes a predetermined region period specifying portion (130) that is adapted to specify a predetermined region period detected by said detection section (120), which detected and specified period is a periodic time variation in a physical state of said predetermined region.

2. The image-generating apparatus (3) according to claim 1, wherein
said displaying image (IG) includes an index, which is provided in association with said summary display portion and which indicates a specific position in the time axis direction of said summary display portion; and
said display image generating section (160) generates said displaying image (IG) to display a dynamic image (MI) at a time point corresponding to the specific position indicated by said index in said dynamic image display portion.

3. The image-generating apparatus (3) according to claim 1, wherein
said predetermined region is a lung field;
said first analysis result indicates exhalation; and said second analysis result indicates inhalation.

4. The image-generating apparatus (3) according to claim 1 or 2, wherein
said predetermined region includes a plurality of predetermined regions;
said detecting section (120) detects a time variation in a physical state of each of the plurality of predetermined regions;
said diagnosis assisting information generating section (150) performs analysis based on the time variation in the physical state of each of the plurality of predetermined regions, and generates an analysis result for each of the plurality of predetermined regions as a plurality of diagnosis assisting information; and
said summary display portion displays said plurality of diagnosis assisting information.

5. The image-generating apparatus (3) according to claim 4, wherein said plurality of regions includes a left lung field and a right lung field.

6. The image-generating apparatus (3) according to claim 1 or 2, wherein said detecting section (120) includes a feature point calculating portion that calculates a feature point in the time variation in the physical state of said predetermined region.

7. The image-generating apparatus (3) according to any one of claims 1 to 6, wherein said detecting section (120) detects the time variation in the physical state of said predetermined region based on said dynamic image (MI).

8. The image-generating apparatus (3) according to any one of claims 1 to 7, wherein said summary display portion displays said first analysis result and said second analysis result in different colors or shading.

9. The image-generating apparatus (3) according to any one of claims 1 to 8, wherein
said dynamic image display portion is a rectangle; and said summary display portion is a long region lying along one side of said dynamic image display portion, its longitudinal direction corresponds to a time axis direction of said diagnosis assisting information.

10. The image-generating apparatus (3) according to any one of claims 1 to 9, wherein
said displaying image (IG) further includes a playback time display portion that displays playback time information corresponding to the display of said dynamic image display portion.

11. The image-generating apparatus (3) according to any one of claims 1 to 10, further comprising a playback time adjustment interface for playback time adjustment of the dynamic image (MI) displayed in said dynamic image display portion.

12. The image-generating apparatus (3) according to any one of claims 1 to 11, further comprising an informing unit that, when the analysis result by said diagnosis assisting information generating section (150) satisfies a predetermined condition, informs the user that said predetermined condition is satisfied.

13. The image-generating apparatus (3) according to claim 7, wherein
said detecting section (120) outputs an image processing result related to presence or absence of a blood flow of said predetermined region based on said dynamic image (MI); and
said display image generating section (160) generates said displaying image (IG) to display said image processing result in synchronization with said dynamic image (MI).

## Patentansprüche

1. Bilderzeugungsvorrichtung (3), umfassend:
einen dynamischen Bilderfassungsbereich (110), der ein dynamisches Bild (MI) erfasst, in dem eine vorgegebene Region eines menschlichen Körpers oder eines Tiers chronologisch aufgenommen wird;
einen Detektionsbereich (120), der eine Zeitabweichung in einem physischen Zustand der vorgegebenen Region detektiert;
einen Bereich (150) zur Erzeugung von diagnose-unterstützenden Informationen, der eine Analyse basierend auf der durch den Detektionsbereich (120) detektierten Zeitabweichung in dem physischen Zustand der vorgegebenen Region durchführt und ein erstes und zweites Analyseergebnis basierend auf der Analyse als diagnose-unterstützende Informationen erzeugt, wobei die diagnose-unterstützenden Informationen ein erstes Analyseergebnis und ein zweites Analyseergebnis basierend auf der Analyse umfassen,
eine Halteeinheit (32), die die diagnose-unterstützenden Informationen in zeitlichem Zusammenhang mit dem dynamischen Bild (MI) hält; und
einen Anzeigebilderzeugungsbereich (160), der einen dynamischen Bildanzeigeabschnitt umfasst, welcher dafür ausgelegt ist, ein Wiedergabebild zu erzeugen, das dafür ausgelegt ist, das dynamische Bild (MI) wiederzugeben, wobei das Anzeigebild (IG) ein Bild ist, das einen Zusammenfassungsanzeigeabschnitt umfasst, welcher dafür ausgelegt ist, einen Fortschrittsbalken (PB) entsprechend dem Fortschritt des Wiedergabebilds anzuzeigen, und wobei das erste Analyseergebnis der Atmungsphase eines rechten Lungenfelds entspricht und das zweite Analyseergebnis der Atmungsphase eines linken Lungenfelds entspricht, und wobei der Fortschrittsbalken (PB) und die Analyseergebnisse in einer integrierten Weise auf dem Fortschrittsbalken angezeigt werden und derartig, dass das erste und das zweite Analyseergebnis als parallele Balken angezeigt werden, um so auf einen Blick in einer Zeitachsenrichtung unterscheidbar zu sein,
wobei der Detektionsbereich (120) einen Abschnitt (130) zum Spezifizieren einer vorgegebenen Regionsperiode umfasst, der dafür ausgelegt ist, eine durch den Detektionsbereich (120) detektierte vorgegebene Regionsperiode zu spezifizieren, wobei die detektierte und spezifizierte Periode eine periodische Zeitabweichung in einem physischen Zustand der vorgegebenen Region ist.

2. Bilderzeugungsvorrichtung (3) nach Anspruch 1, wobei
das Anzeigebild (IG) einen Index umfasst, der in Zusammenhang mit dem Zusammenfassungsanzeigeabschnitt bereitgestellt wird und der eine bestimmte Position in der Zeitachsenrichtung des Zusammenfassungsanzeigeabschnitts angibt; und
der Anzeigebilderzeugungsbereich (160) das Anzeigebild (IG) erzeugt, um ein dynamisches Bild (MI) zu einem Zeitpunkt anzuzeigen, der der durch den Index in dem dynamischen Bildanzeigebereich angegebenen bestimmten Position entspricht.

3. Bilderzeugungsvorrichtung (3) nach Anspruch 1, wobei
die vorgegebene Region ein Lungenfeld ist;
das erste Analyseergebnis Ausatmung angibt; und
das zweite Analyseergebnis Einatmung angibt.

4. Bilderzeugungsvorrichtung (3) nach Anspruch 1 oder 2, wobei die vorgegebene Region eine Vielzahl von vorgegebenen Regionen umfasst;
der Detektionsbereich (120) eine Zeitabweichung in einem physischen Zustand von jeder der Vielzahl von vorgegebenen Regionen detektiert;
der Bereich (150) zur Erzeugung diagnose-unterstützender Informationen eine Analyse basierend auf der Zeitabweichung in dem physischen Zustand von jeder der Vielzahl von vorgegebenen Regionen durchführt und ein Analyseergebnis für jede von der Vielzahl von vorgegebenen Regionen als eine Vielzahl von diagnose-unterstützenden Informationen erzeugt; und
der Zusammenfassungsanzeigeabschnitt die Vielzahl von diagnose-unterstützenden Informationen anzeigt.

5. Bilderzeugungsvorrichtung (3) nach Anspruch 4, wobei die Vielzahl von Regionen ein linkes Lungenfeld und ein rechtes Lungenfeld umfasst.

6. Bilderzeugungsvorrichtung (3) nach Anspruch 1 oder 2, wobei der Detektionsbereich (120) einen Merkmalspunktberechnungsabschnitt umfasst, der einen Merkmalspunkt in der Zeitabweichung in dem physischen Zustand der vorgegebenen Region berechnet.

7. Bilderzeugungsvorrichtung (3) nach einem der Ansprüche 1 bis 6, wobei der Detektionsbereich (120) die Zeitabweichung in dem physischen Zustand der vorgegebenen Region basierend auf dem dynamischen Bild (MI) detektiert.

8. Bilderzeugungsvorrichtung (3) nach einem der Ansprüche 1 bis 7, wobei der Zusammenfassungsanzeigeabschnitt das erste Analyseergebnis und das zweite Analyseergebnis in verschiedenen Farben oder Schattierungen anzeigt.

9. Bilderzeugungsvorrichtung (3) nach einem der Ansprüche 1 bis 8, wobei
der dynamische Bildanzeigebereich ein Rechteck ist; und
der Zusammenfassungsanzeigeabschnitt eine lange Region ist, die entlang einer Seite des dynamischen Bildanzeigeabschnitts liegt, wobei ihre Längsrichtung der Zeitachsenrichtung der diagnose-unterstützenden Informationen entspricht.

10. Bilderzeugungsvorrichtung (3) nach einem der Ansprüche 1 bis 9, wobei
das Anzeigebild (IG) weiter einen Wiedergabezeitanzeigeabschnitt umfasst, der die Wiedergabezeitinformationen entsprechend der Anzeige des dynamischen Bildanzeigeabschnitts anzeigt.

11. Bilderzeugungsvorrichtung (3) nach einem der Ansprüche 1 bis 10, weiter umfassend eine Wiedergabezeiteinstellungsschnittstelle zum Einstellen der Wiedergabezeit des dynamischen Bilds (MI), das in dem dynamischen Bildanzeigeabschnitt angezeigt wird.

12. Bilderzeugungsvorrichtung (3) nach einem der Ansprüche 1 bis 11, weiter umfassend eine Informationseinheit, die, wenn das Analyseergebnis durch den diagnose-unterstützende Informationen erzeugenden Bereich (150) eine vorgegebene Bedingung erfüllt, den Benutzer informiert, dass die vorgegebene Bedingung erfüllt ist.

13. Bilderzeugungsvorrichtung (3) nach Anspruch 7, wobei
der Detektionsbereich (120) ein Bildverarbeitungsergebnis in Bezug auf die Anwesenheit oder Abwesenheit einer Blutströmung der vorgegebenen Region basierend auf dem dynamischen Bild (MI) ausgibt; und
der Anzeigebilderzeugungsbereich (160) das Anzeigebild (IG) erzeugt, um das Bildverarbeitungsergebnis in Synchronisation mit dem dynamischen Bild (MI) anzuzeigen.

## Revendications

1. Appareil de production d'image (3) comprenant :
une section d'acquisition d'image dynamique (110) qui acquiert une image dynamique (MI) dans laquelle une région prédéterminée d'un corps humain ou d'un animal est capturée chronologiquement ;
une section de détection (120) qui détecte une variation temporelle dans un état physique de ladite région prédéterminée ;
une section de génération d'informations d'aide au diagnostic (150) qui effectue une analyse sur la base de la variation temporelle dans l'état physique de ladite région prédéterminée détectée par ladite section de détection (120), et génère un premier et un second résultat d'analyse sur la base de ladite analyse en tant qu'informations d'aide au diagnostic, lesdites informations d'aide au diagnostic comprenant un premier résultat d'analyse et un second résultat d'analyse basés sur ladite analyse,
une unité de conservation (32) qui conserve lesdites informations d'aide au diagnostic en association temporelle avec ladite image dynamique (MI) ; et
une section de génération d'image d'affichage (160) qui comprend une partie d'affichage d'image dynamique adaptée pour générer une image de lecture adaptée pour lire ladite image dynamique (MI), ladite image d'affichage (IG) est une image qui comprend une partie d'affichage de synthèse qui est adaptée pour afficher une barre de progression (PB) correspondant à la progression de ladite image de lecture et dans lequel le premier résultat d'analyse correspond à la phase de respiration d'un champ pulmonaire droit et le second résultat d'analyse correspond à la phase de respiration d'un champ pulmonaire gauche et dans lequel la barre de progression (PB) et lesdits résultats d'analyse sont affichés de façon intégrée sur ladite barre de progression et de telle sorte que lesdits premier et second résultats d'analyse sont affichés en tant que barres parallèles de façon à être différenciables d'un coup d'oeil dans une direction d'axe temporel,
dans lequel ladite section de détection (120) comprend une partie de spécification de période de région prédéterminée (130) qui est adaptée pour spécifier une période de région prédéterminée détectée par ladite section de détection (120), laquelle période détectée et spécifiée est une variation temporelle périodique dans un état physique de ladite région prédéterminée.

2. Appareil de production d'image (3) selon la revendication 1, dans lequel
ladite image d'affichage (IG) comprend un index, qui est fourni en association avec ladite partie d'affichage de synthèse et qui indique une position spécifique dans la direction de l'axe temporel de ladite partie d'affichage de synthèse ; et
ladite section de génération d'image d'affichage (160) génère ladite image d'affichage (IG) pour afficher une image dynamique (MI) au niveau d'un point temporel correspondant à la position spécifique indiquée par ledit index dans ladite partie d'affichage d'image dynamique.

3. Appareil de production d'image (3) selon la revendication 1, dans lequel ladite région prédéterminée est un champ pulmonaire ;
ledit premier résultat d'analyse indique une expiration ; et
ledit second résultat d'analyse indique une inspiration.

4. Appareil de production d'image (3) selon la revendication 1 ou 2, dans lequel
ladite région prédéterminée comprend une pluralité de régions prédéterminées ;
ladite section de détection (120) détecte une variation temporelle dans un état physique de chacune de la pluralité de régions prédéterminées ;
ladite section de génération d'informations d'aide au diagnostic (150) effectue une analyse sur la base de la variation temporelle dans l'état physique de chacune de la pluralité de régions prédéterminées, et génère un résultat d'analyse pour chacune de la pluralité de régions prédéterminée en tant qu'une pluralité d'informations d'aide au diagnostic ; et
ladite partie d'affichage de synthèse affiche ladite pluralité d'informations d'aide au diagnostic.

5. Appareil de production d'image (3) selon la revendication 4, dans lequel ladite pluralité de régions comprend un champ pulmonaire gauche et un champ pulmonaire droit.

6. Appareil de production d'image (3) selon la revendication 1 ou 2, dans lequel ladite section de détection (120) comprend une partie de calcul de point caractéristique qui calcule un point caractéristique dans la variation temporelle dans l'état physique de ladite région prédéterminée.

7. Appareil de production d'image (3) selon l'une quelconque des revendications 1 à 6, dans lequel ladite section de détection (120) détecte la variation temporelle dans l'état physique de ladite région prédéterminée sur la base de ladite image dynamique (MI).

8. Appareil de production d'image (3) selon l'une quelconque des revendications 1 à 7, dans lequel ladite partie d'affichage de synthèse affiche ledit premier résultat d'analyse et ledit second résultat d'analyse dans différents dégradés ou couleurs.

9. Appareil de production d'image (3) selon l'une quelconque des revendications 1 à 8, dans lequel
ladite partie d'affichage d'image dynamique est un rectangle ; et
ladite partie d'affichage de synthèse est une région longue reposant le long d'un côté de ladite partie d'affichage d'image dynamique, sa direction longitudinale correspond à une direction d'axe temporel desdites informations d'aide au diagnostic.

10. Appareil de production d'image (3) selon l'une quelconque des revendications 1 à 9, dans lequel
ladite image d'affichage (IG) comprend en outre une partie d'affichage temporel de lecture qui affiche des informations temporelles de lecture correspondant à l'affichage de ladite partie d'affichage d'image dynamique.

11. Appareil de production d'image (3) selon l'une quelconque des revendications 1 à 10, comprenant en outre une interface d'ajustement temporel de lecture pour l'ajustement temporel de lecture de l'image dynamique (MI) affichée dans ladite partie d'affichage d'image dynamique.

12. Appareil de production d'image (3) selon l'une quelconque des revendications 1 à 11, comprenant en outre une unité d'information qui, lorsque le résultat d'analyse fourni par ladite section de génération d'informations d'aide au diagnostic (150) satisfait à une condition prédéterminée, informe l'utilisateur que ladite condition prédéterminée est satisfaite.

13. Appareil de production d'image (3) selon la revendication 7, dans lequel
ladite section de détection (120) produit un résultat de traitement d'image associé à la présence ou à l'absence d'un flux sanguin de ladite région prédéterminée sur la base de ladite image dynamique (MI) ; et
ladite section de génération d'image d'affichage (160) génère ladite image d'affichage (IG) pour afficher ledit résultat de traitement d'image en synchronisation avec ladite image dynamique (MI).
